# EUROPEAN PATENT APPLICATION

(11) **EP 4 299 568 A1**
(43) Date of publication of application: **03.01.2024**
(21) Application number: 22767466.0
(22) Date of filing: 08.03.2022
(51) Int. Cl.: C07D 307/79, C07D 407/04, C07D 405/04, C07D 407/14, C07D 409/04, C07D 333/54, C07D 487/04, C07D 407/12, C07F 7/08, C07F 5/02

(54) **ORGANIC LIGHT-EMITTING COMPOUND AND ORGANIC LIGHT-EMITTING DEVICE COMPRISING SAME**

(30) Priority: 08.03.2021 KR 20210030258
(71) Applicant: SFC Co., Ltd., Cheongju-si, Chungcheongbuk-do 28122 (KR)
(72) Inventor: LEE, Se-jin, Cheongju-si Chungcheongbuk-do 28122 (KR); KIM, Si-in, Cheongju-si Chungcheongbuk-do 28122 (KR); PARK, Seok-bae, Cheongju-si Chungcheongbuk-do 28122 (KR); KIM, Hee-dae, Cheongju-si Chungcheongbuk-do 28122 (KR); CHOI, Yeong-tae, Cheongju-si Chungcheongbuk-do 28122 (KR); LEE, Seung-soo, Cheongju-si Chungcheongbuk-do 28122 (KR); KIM, Ji-yung, Cheongju-si Chungcheongbuk-do 28122 (KR); KIM, Kyeong-hyeon, Cheongju-si Chungcheongbuk-do 28122 (KR); KIM, Kyung-tae, Cheongju-si Chungcheongbuk-do 28122 (KR); KIM, Myeong-jun, Cheongju-si Chungcheongbuk-do 28122 (KR); LEE, Tae-gyun, Cheongju-si Chungcheongbuk-do 28122 (KR); KIM, Joon-ho, Cheongju-si Chungcheongbuk-do 28122 (KR)
(74) Representative: Grosse, Felix Christopher
(86) International application number: PCT/KR2022/003252
(87) International publication number: WO 2022/191570

(57) **Abstract**

The present invention relates to a high-efficiency organic light-emitting device having excellent light-emitting characteristics in light-emitting efficiency, etc. by employing an anthracene derivative compound having a characteristic structure as a host in a light-emitting layer. By using the high-efficiency organic light-emitting device, according to the present invention, the organic light-emitting device can be utilized for various display devices such as flat panel, flexible, and wearable displays as well as a lighting device.

## Description

### Technical Field

The present invention relates to an anthracene derivative with a specific structure and a highly efficient organic light emitting device that includes a light emitting layer employing the anthracene derivative as a host, achieving significantly improved external quantum efficiency.

### Background Art

Organic light emitting devices are self-luminous devices in which electrons injected from an electron injecting electrode (cathode) recombine with holes injected from a hole injecting electrode (anode) in a light emitting layer to form excitons, which emit light while releasing energy. Such organic light emitting devices have the advantages of low driving voltage, high luminance, large viewing angle, and short response time and can be applied to full-color light emitting flat panel displays. Due to these advantages, organic light emitting devices have received attention as next-generation light sources.

The above characteristics of organic light emitting devices are achieved by structural optimization of organic layers of the devices and are supported by stable and efficient materials for the organic layers, such as hole injecting materials, hole transport materials, light emitting materials, electron transport materials, electron injecting materials, and electron blocking materials. However, more research still needs to be done to develop structurally optimized structures of organic layers for organic light emitting devices and stable and efficient materials for organic layers of organic light emitting devices.

Particularly, for maximum efficiency in a light emitting layer, an appropriate combination of energy band gaps of a host and a dopant is required such that holes and electrons migrate to the dopant through stable electrochemical paths to form excitons.

### Detailed Description of the Invention

### Problems to be Solved by the Invention

Accordingly, the present invention is intended to provide a host material with a specific structure that is employed in a light emitting layer of an organic light emitting device and a highly efficient organic light emitting device with significantly improved luminous efficiency that employs the host material.

### Means for Solving the Problems

One aspect of the present invention provides an anthracene derivative that is employed as a host compound in an organic layer, preferably a light emitting layer of a device wherein the anthracene derivative is represented by Formula A:

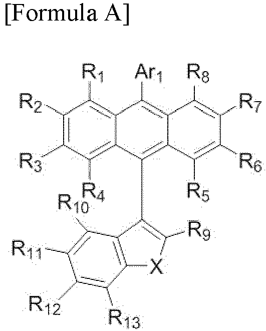

The specific structure of Formula A, definitions of the substituents in Formula A, and specific compounds that can be represented by Formula A are described below.

Another aspect of the present invention provides an organic light emitting device including a first electrode, a second electrode opposite to the first electrode, and a light emitting layer interposed between the first and second electrodes wherein the light emitting layer includes the anthracene derivative represented by Formula A as a host.

According to one embodiment of the present invention, significantly improved luminous efficiency and high efficiency of the organic light emitting device are ensured when the light emitting layer employs the anthracene derivative represented by Formula A as a host and a compound selected from those represented by Formulas D-1 and D-2 as a dopant:

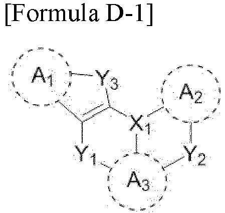

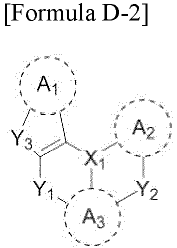

The specific structures of Formulas D-1 and D-2, definitions of the substituents in Formulas D-1 and D-2, and specific compounds that can be represented by Formulas D-1 and D-2 are described below.

### Effects of the Invention

The organic light emitting device of the present invention includes a light emitting layer employing the anthracene derivative with a specific structure as a host. The use of the host ensures excellent luminescent properties (including high luminous efficiency) and high efficiency of the organic light emitting device. Due to these advantages, the organic light emitting device of the present invention can find useful applications in not only lighting systems but also a variety of displays, including flat panel displays, flexible displays, and wearable displays.

### Best Mode for Carrying out the Invention

The present invention will now be described in more detail.

One aspect of the present invention is directed to an anthracene derivative represented by Formula A: 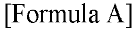 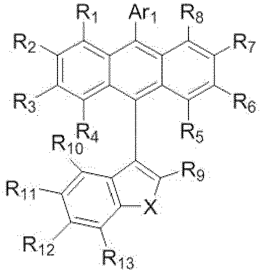 wherein Ar₁ is selected from substituted or unsubstituted C₆-C₅₀ aryl, substituted or unsubstituted C₂-C₅₀ heteroaryl, and substituted or unsubstituted C₃-C₅₀ mixed aliphatic-aromatic cyclic groups, R₁ to R₁₃ are the same as or different from each other and are each independently selected from hydrogen, deuterium, substituted or unsubstituted C₁-C₃₀ alkyl, substituted or unsubstituted C₆-C₅₀ aryl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₂-C₅₀ heteroaryl, substituted or unsubstituted C₂-C₃₀ heterocycloalkyl, substituted or unsubstituted C₁-C₃₀ alkoxy, substituted or unsubstituted C₆-C₅₀ aryloxy, substituted or unsubstituted C₁-C₃₀ alkylthioxy, substituted or unsubstituted C₆-C₅₀ arylthioxy, substituted or unsubstituted amine, substituted or unsubstituted silyl, substituted or unsubstituted germanium, substituted or unsubstituted C₃-C₅₀ mixed aliphatic-aromatic cyclic groups, cyano, and halogen, with the proviso that R₁₂ is other than aryl, and X is an oxygen (O) or sulfur (S) atom.

The anthracene derivative of the present invention has a structure in which the C-9 position of anthracene is connected to the C-3 position of benzofuran (thiophene). The use of the anthracene derivative as a host compound in a light emitting layer of an organic light emitting device makes the device highly efficient.

As used herein, the term "substituted" in the definitions of Ar₁ and R₁ to R₁₃ in Formula A indicates substitution with one or more substituents selected from deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₃₀ aryl, C₇-C₃₀ arylalkyl, C₇-C₃₀ alkylaryl, C₂-C₃₀ heteroaryl, C₂-C₃₀ heteroarylalkyl, C₁-C₂₄ alkoxy, C₁-C₂₄ amine, C₁-C₂₄ silyl, C₁-C₂₄ germanium, C₆-C₃₀ aryloxy, C₃-C₃₀ mixed aliphatic-aromatic cyclic groups, boron, aluminum, phosphoryl, hydroxyl, selenium, tellurium, nitro, and halogen. One or more hydrogen atoms in each of the substituents are optionally replaced by deuterium atoms.

The present invention is also directed to an organic light emitting device including a first electrode, a second electrode opposite to the first electrode, and one or more organic layers interposed between the first and second electrodes wherein one of the organic layers, preferably a light emitting layer includes the anthracene derivative represented by Formula A.

According to one embodiment of the present invention, the light emitting layer of the organic light emitting device may include, as a host, the anthracene derivative represented by Formula A and, as a dopant, a compound selected from those represented by Formulas D-1 and D-2: 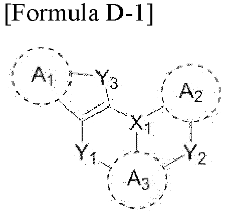 wherein X₁ is selected from B, P=O, and P=S, Y₁ to Y₃ are each independently selected from NR₂₁, CR₂₂R₂₃, O, S, Se, and SiR₂₄R₂₅, A₁ to A₃ are each independently selected from substituted or unsubstituted C₆-C₅₀ aromatic hydrocarbon rings, substituted or unsubstituted C₂-C₅₀ aromatic heterocyclic rings, substituted or unsubstituted C₃-C₃₀ aliphatic rings, and substituted or unsubstituted C₃-C₃₀ mixed aliphatic-aromatic rings, and R₂₁ to R₂₅ are the same as or different from each other and are each independently selected from hydrogen, deuterium, substituted or unsubstituted C₁-C₃₀ alkyl, substituted or unsubstituted C₂-C₃₀ alkenyl, substituted or unsubstituted C₂-C₃₀ alkynyl, substituted or unsubstituted C₆-C₅₀ aryl, substituted or unsubstituted C₃-C₃₀ cycloalkyl, substituted or unsubstituted C₃-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₆-C₃₀ arylthioxy, substituted or unsubstituted amine, substituted or unsubstituted silyl, substituted or unsubstituted C₃-C₂₀ mixed aliphatic-aromatic cyclic groups, nitro, cyano, and halogen, with the proviso that each of R₂₁ to R₂₅ is optionally bonded to one or more of the rings A₁ to A₃ to form an alicyclic or aromatic monocyclic or polycyclic ring, the substituents of each of the rings A₁ to A₃ are optionally linked to each other to form an alicyclic or aromatic monocyclic or polycyclic ring, R₂₂ and R₂₃ are optionally linked to each other to form an alicyclic or aromatic monocyclic or polycyclic ring, and R₂₄ and R₂₅ are optionally linked to each other to form an alicyclic or aromatic monocyclic or polycyclic ring, and 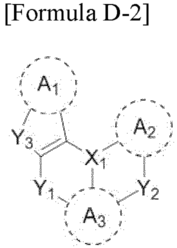 wherein X₁, Y₁ to Y₃, and A₁ to A₃ are as defined in Formula D-1.

As used herein, the term "substituted" in the definitions of A₁ to A₃ and R₂₁ to R₂₅ indicates substitution with one or more substituents selected from deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₃₀ aryl, C₇-C₃₀ arylalkyl, C₇-C₃₀ alkylaryl, C₂-C₃₀ heteroaryl, C₂-C₃₀ heteroarylalkyl, C₁-C₂₄ alkoxy, C₁-C₂₄ amine, C₁-C₂₄ silyl, C₆-C₃₀ aryloxy, C₃-C₃₀ mixed aliphatic-aromatic cyclic groups, boron, aluminum, phosphoryl, hydroxyl, selenium, tellurium, nitro, and halogen. One or more hydrogen atoms in each of the substituents are optionally replaced by deuterium atoms.

The content of the dopant in the light emitting layer is typically in the range of about 0.01 to about 20 parts by weight, based on about 100 parts by weight of the host but is not limited to this range.

The light emitting layer may further include one or more hosts other than the host represented by Formula A and one or more dopant materials other than the dopant represented by one of Formulas D-1 and D-2.

In the "substituted or unsubstituted C₁-C₃₀ alkyl", "substituted or unsubstituted C₆-C₅₀ aryl", etc., the number of carbon atoms in the alkyl or aryl group indicates the number of carbon atoms constituting the unsubstituted alkyl or aryl moiety without considering the number of carbon atoms in the substituent(s). For example, a phenyl group substituted with a butyl group at the para-position corresponds to a C₆ aryl group substituted with a C₄ butyl group.

As used herein, the expression "bonded to an adjacent group to form a ring" means that the corresponding group combines with an adjacent group to form a substituted or unsubstituted alicyclic or aromatic ring and the term "adjacent substituent" may mean a substituent on an atom directly attached to an atom substituted with the corresponding substituent, a substituent disposed sterically closest to the corresponding substituent or another substituent on an atom substituted with the corresponding substituent. For example, two substituents substituted at the ortho position of a benzene ring or two substituents on the same carbon in an aliphatic ring may be considered "adjacent" to each other.

In the present invention, the alkyl groups may be straight or branched. Specific examples of the alkyl groups include, but are not limited to, methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 2-methylpentyl, 4-methylhexyl, and 5-methylhexyl groups.

The alkenyl group is intended to include straight and branched ones and may be optionally substituted with one or more other substituents. The alkenyl group may be specifically a vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, stilbenyl or styrenyl group but is not limited thereto.

The alkynyl group is intended to include straight and branched ones and may be optionally substituted with one or more other substituents. The alkynyl group may be, for example, ethynyl or 2-propynyl but is not limited thereto.

The cycloalkenyl group is a non-aromatic cyclic unsaturated hydrocarbon group having one or more carbon-carbon double bonds. The cycloalkenyl group may be, for example, cyclopropenyl, cyclobutenyl, cyclopentenyl, cyclohexenyl, cycloheptenyl, 1,3-cyclohexadienyl, 1,4-cyclohexadienyl, 2,4-cycloheptadienyl or 1,5-cyclooctadienyl but is not limited thereto.

The aromatic hydrocarbon rings or aryl groups may be monocyclic or polycyclic ones. Examples of the monocyclic aryl groups include, but are not limited to, phenyl, biphenyl, terphenyl, and stilbenyl groups. Examples of the polycyclic aryl groups include naphthyl, anthracenyl, phenanthrenyl, pyrenyl, perylenyl, tetracenyl, chrysenyl, fluorenyl, acenaphathcenyl, triphenylene, and fluoranthrene groups but the scope of the present invention is not limited thereto.

The aromatic heterocyclic rings or heteroaryl groups refer to aromatic groups containing one or more heteroatoms. Examples of the aromatic heterocyclic rings or heteroaryl groups include, but are not limited to, thiophene, furan, pyrrole, imidazole, thiazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, and phenothiazinyl groups.

The aliphatic hydrocarbon rings or cycloalkyl groups refer to non-aromatic rings consisting only of carbon and hydrogen atoms. The aliphatic hydrocarbon ring is intended to include monocyclic and polycyclic ones and may be optionally substituted with one or more other substituents. As used herein, the term "polycyclic" means that the aliphatic hydrocarbon ring may be directly attached or fused to one or more other cyclic groups. The other cyclic groups may be aliphatic hydrocarbon rings and other examples thereof include aliphatic heterocyclic, aryl, and heteroaryl groups. Specific examples of the aliphatic hydrocarbon rings include, but are not limited to, cycloalkyl groups such as cyclopropyl, cyclobutyl, cyclopentyl, adamantyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl, cycloalkanes such as cyclohexane and cyclopentane, and cycloalkenes such as cyclohexene and cyclobutene.

The aliphatic heterocyclic rings or heterocycloalkyl groups refer to aliphatic rings containing one or more heteroatoms such as O, S, Se, N, and Si. The aliphatic heterocyclic ring is intended to include monocyclic or polycyclic ones and may be optionally substituted with one or more other substituents. As used herein, the term "polycyclic" means that the aliphatic heterocyclic ring such as heterocycloalkyl, heterocycloalkane or heterocycloalkene may be directly attached or fused to one or more other cyclic groups. The other cyclic groups may be aliphatic heterocyclic rings and other examples thereof include aliphatic hydrocarbon rings, aryl groups, and heteroaryl groups.

The mixed aliphatic-aromatic cyclic groups refer to structures in which at least one aliphatic ring and at least one aromatic ring are linked or fused together and which are overall non-aromatic. The mixed aliphatic-aromatic polycyclic rings may contain one or more heteroatoms selected from N, O, P, and S other than carbon atoms (C).

The alkoxy group may be specifically a methoxy, ethoxy, propoxy, isobutyloxy, sec-butyloxy, pentyloxy, iso-amyloxy or hexyloxy group but is not limited thereto.

The silyl group is intended to include -SiH₃, alkylsilyl, arylsilyl, alkylarylsilyl, arylheteroarylsilyl, and heteroaryl silyl. The arylsilyl refers to a silyl group obtained by substituting one, two or three of the hydrogen atoms in -SiH₃ with aryl groups. The alkylsilyl refers to a silyl group obtained by substituting one, two or three of the hydrogen atoms in -SiH₃ with alkyl groups. The alkylarylsilyl refers to a silyl group obtained by substituting one of the hydrogen atoms in -SiH₃ with an alkyl group and the other two hydrogen atoms with aryl groups or substituting two of the hydrogen atoms in -SiH₃ with alkyl groups and the remaining hydrogen atom with an aryl group. The arylheteroaryl silyl refers to a silyl group obtained by substituting one of the hydrogen atoms in -SiH₃ with an aryl group and the other two hydrogen atoms with heteroaryl groups or substituting two of the hydrogen atoms in -SiH₃ with aryl groups and the remaining hydrogen atom with a heteroaryl group. The heteroarylsilyl refers to a silyl group obtained by substituting one, two or three of the hydrogen atoms in -SiH₃ with heteroaryl groups. The arylsilyl group may be, for example, substituted or unsubstituted monoarylsilyl, substituted or unsubstituted diarylsilyl, or substituted or unsubstituted triarylsilyl. The same applies to the alkylsilyl and heteroarylsilyl groups.

Each of the aryl groups in the aryl silyl, heteroarylsilyl, and arylheteroarylsilyl groups may be a monocyclic or polycyclic one. Each of the heteroaryl groups in the arylsilyl, heteroarylsilyl, and arylheteroarylsilyl groups may be a monocyclic or polycyclic one.

Specific examples of the silyl groups include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl. One or more of the hydrogen atoms in each of the silyl groups may be substituted with the substituents mentioned in the aryl groups.

The amine group is intended to include -NH₂, alkylamine, arylamine, arylheteroarylamine, and heteroarylamine. The arylamine refers to an amine group obtained by substituting one or two of the hydrogen atoms in -NH₂ with aryl groups. The alkylamine refers to an amine group obtained by substituting one or two of the hydrogen atoms in -NH₂ with alkyl groups. The alkylarylamine refers to an amine group obtained by substituting one of the hydrogen atoms in -NH₂ with an alkyl group and the other hydrogen atom with an aryl group. The arylheteroarylamine refers to an amine group obtained by substituting one of the hydrogen atoms in -NH₂ with an aryl group and the other hydrogen atom with a heteroaryl group. The heteroarylamine refers to an amine group obtained by substituting one or two of the hydrogen atoms in -NH₂ with heteroaryl groups. The arylamine may be, for example, substituted or unsubstituted monoarylamine, substituted or unsubstituted diarylamine, or substituted or unsubstituted triarylamine. The same applies to the alkylamine and heteroarylamine groups.

Each of the aryl groups in the arylamine, heteroarylamine, and arylheteroarylamine groups may be a monocyclic or polycyclic one. Each of the heteroaryl groups in the arylamine, heteroarylamine, and arylheteroarylamine groups may be a monocyclic or polycyclic one.

The germanium group is intended to include -GeH₃, alkylgermanium, aryl germanium, heteroarylgermanium, alkylarylgermanium, alkylheteroarylgermanium, and arylheteroarylgermanium. The definitions of the substituents in the germanium groups follow those described for the silyl groups, except that the silicon (Si) atom in each silyl group is changed to a germanium (Ge) atom.

Specific examples of the germanium groups include trimethylgermane, triethylgermane, triphenylgermane, trimethoxygermane, dimethoxyphenylgermane, diphenylmethylgermane, diphenylvinylgermane, methylcyclobutylgermane, and dimethylfurylgermane. One or more of the hydrogen atoms in each of the germanium groups may be substituted with the substituents mentioned in the aryl groups.

The cycloalkyl, aryl, and heteroaryl groups in the cycloalkyloxy, aryloxy, heteroaryloxy, cycloalkylthioxy, arylthioxy, and heteroarylthioxy groups are the same as those exemplified above. Specific examples of the aryloxy groups include, but are not limited to, phenoxy, p-tolyloxy, m-tolyloxy, 3,5-dimethylphenoxy, 2,4,6-trimethylphenoxy, p-tert-butylphenoxy, 3-biphenyloxy, 4-biphenyloxy, 1-naphthyloxy, 2-naphthyloxy, 4-methyl-1-naphthyloxy, 5-methyl-2-naphthyloxy, 1-anthryloxy, 2-anthryloxy, 9-anthryloxy, 1-phenanthryloxy, 3-phenanthryloxy, and 9-phenanthryloxy groups. Specific examples of the arylthioxy groups include, but are not limited to, phenylthioxy, 2-methylphenylthioxy, and 4-tert-butylphenylthioxy groups.

The halogen group may be, for example, fluorine, chlorine, bromine or iodine.

According to one embodiment of the present invention, the anthracene derivative represented by Formula A may be selected from the following compounds 1 to 60:

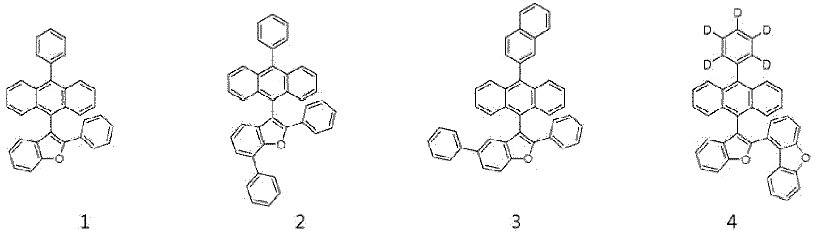

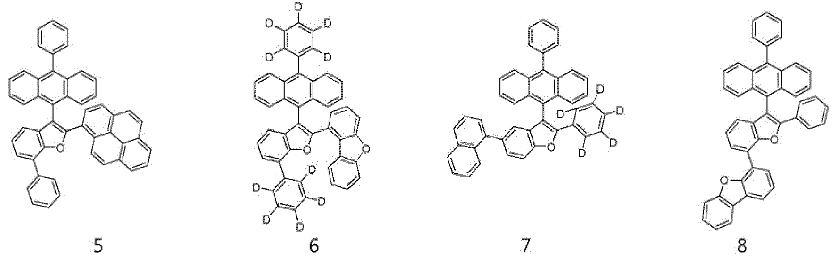

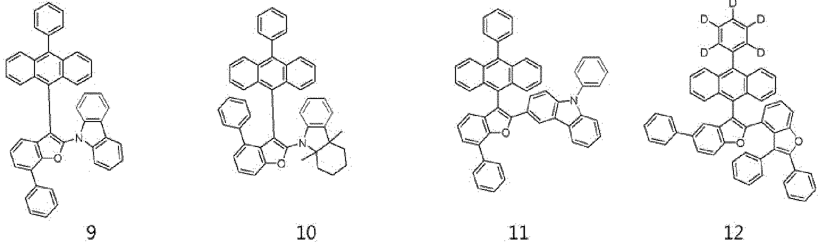

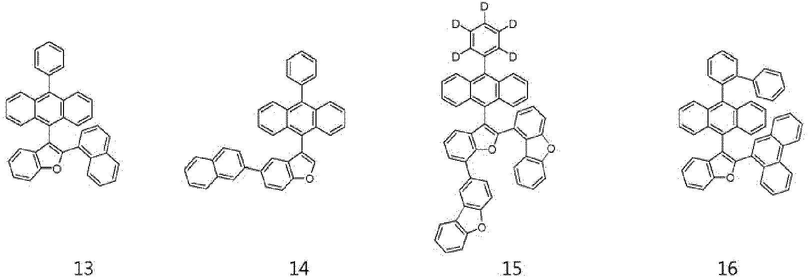

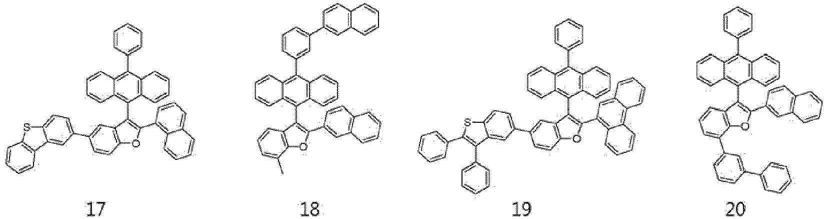

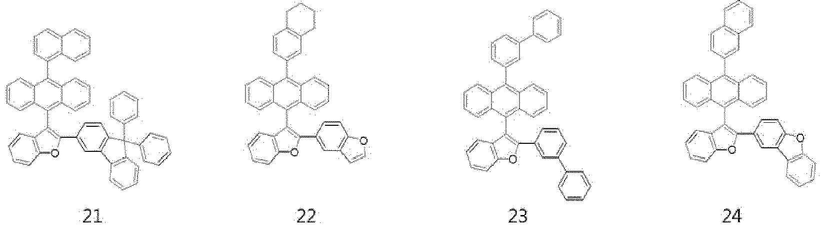

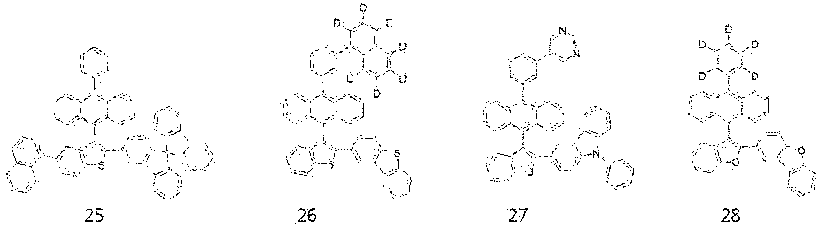

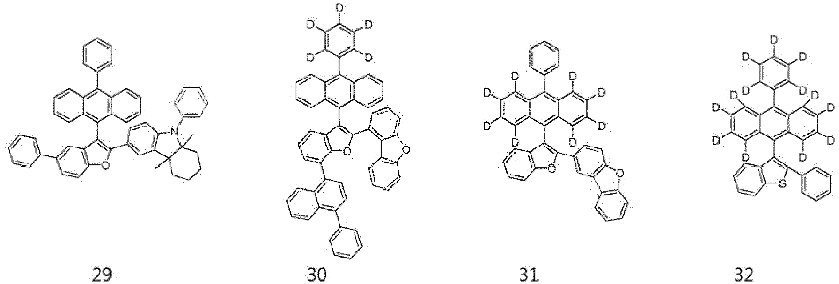

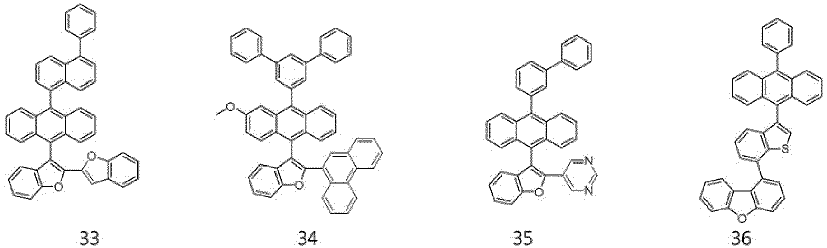

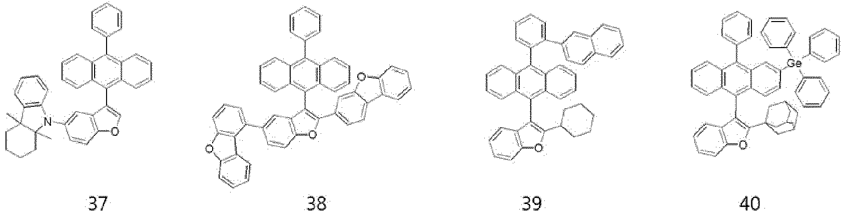

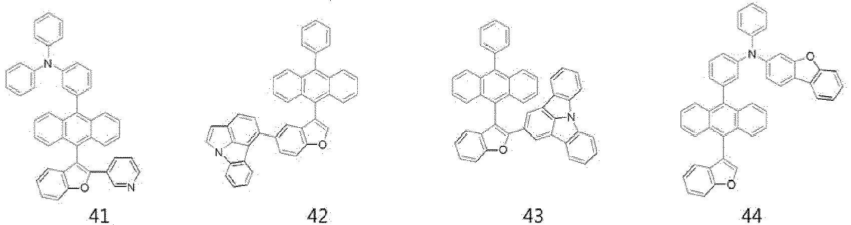

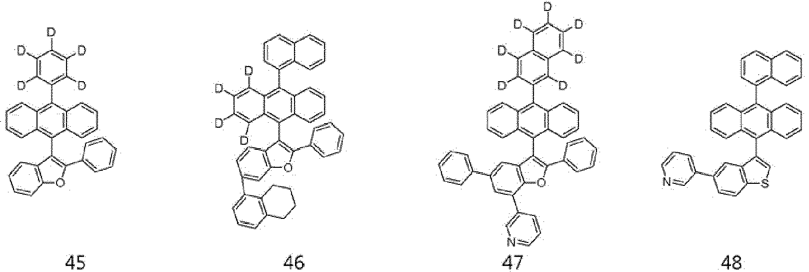

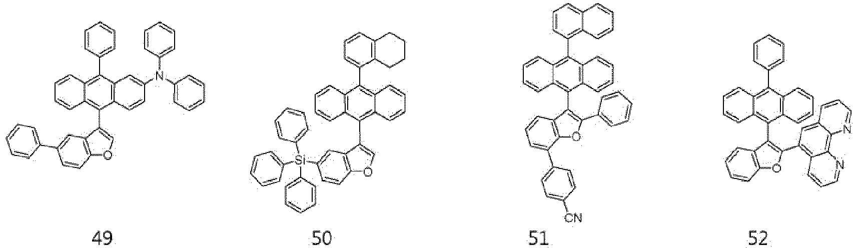

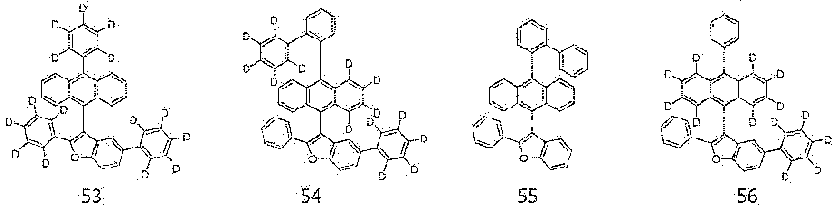

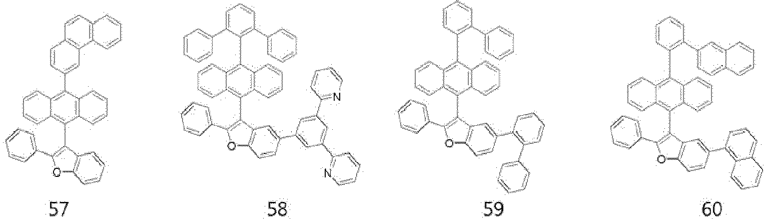

However, these compounds are not intended to limit the scope of Formula A.

According to one embodiment of the present invention, the compounds represented by Formulas D-1 and D-2 may be selected from the following compounds D-101 to D-199:

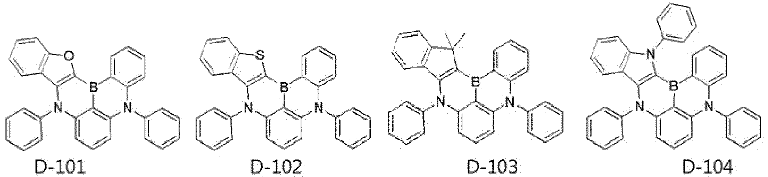

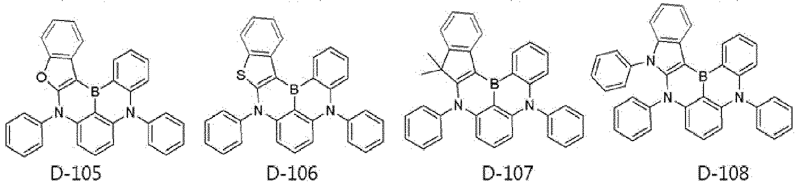

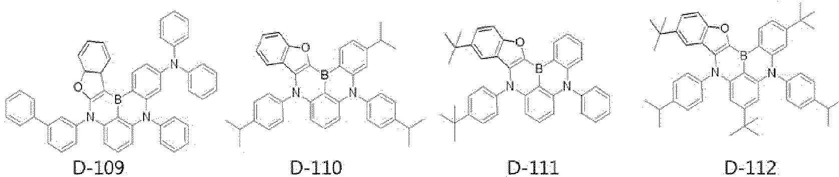

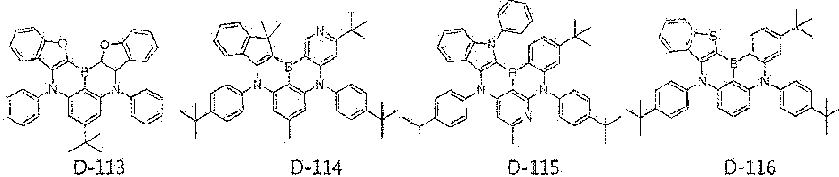

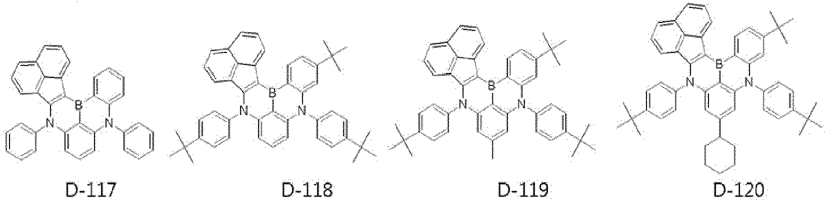

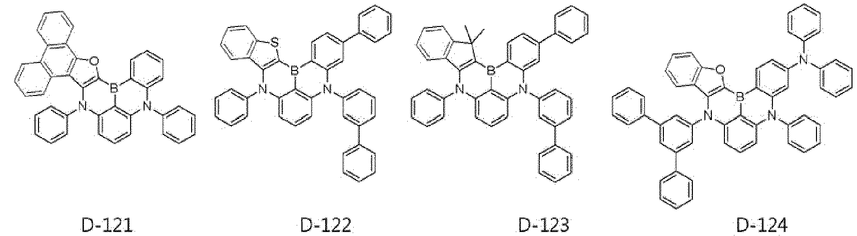

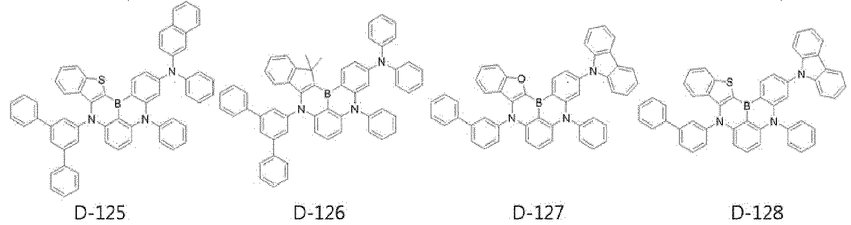

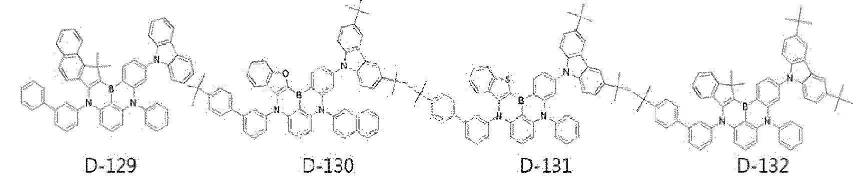

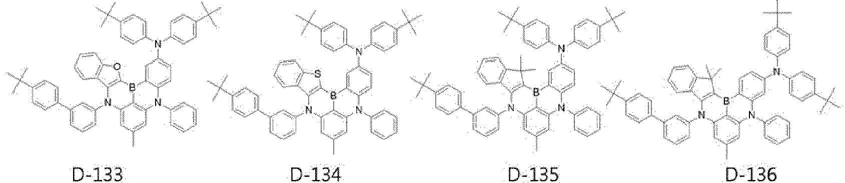

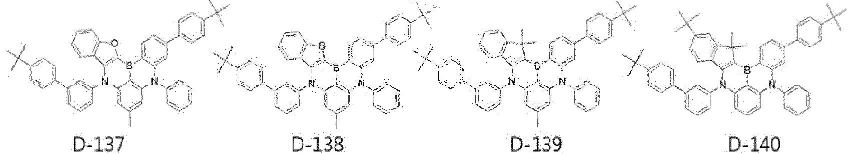

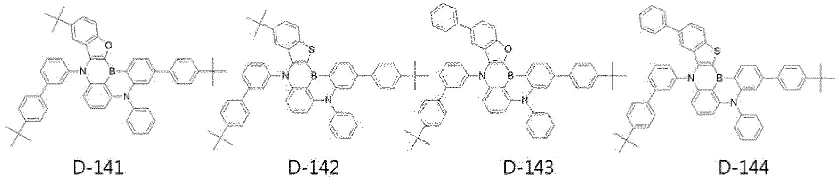

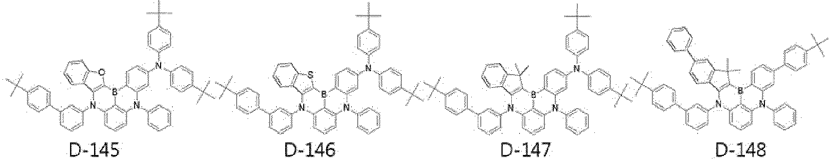

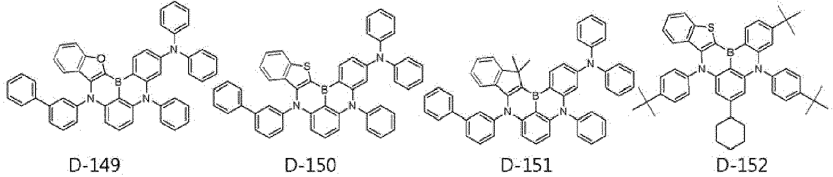

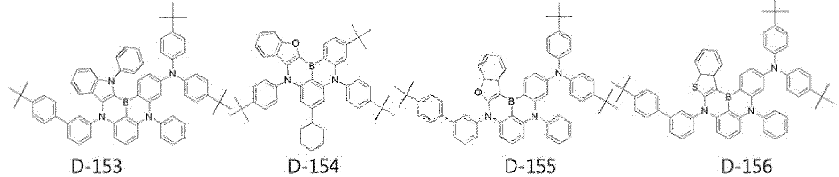

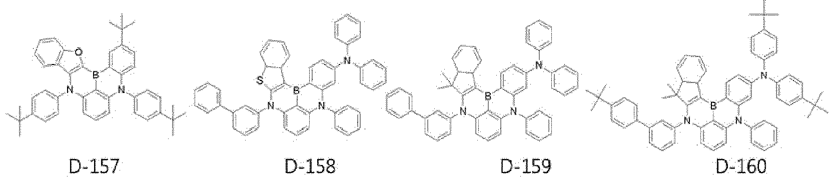

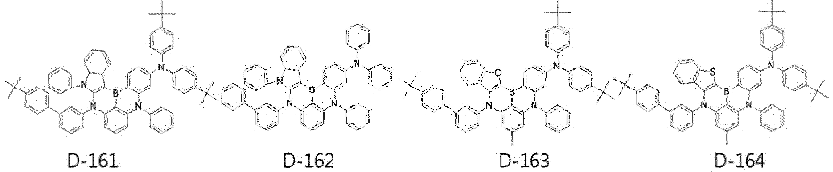

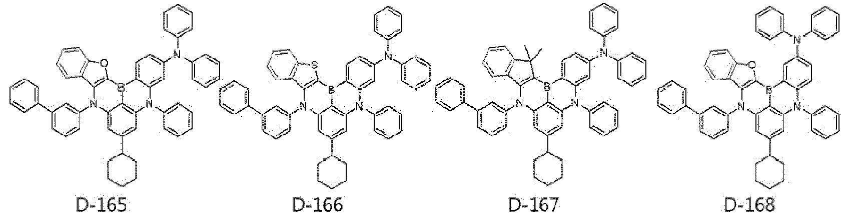

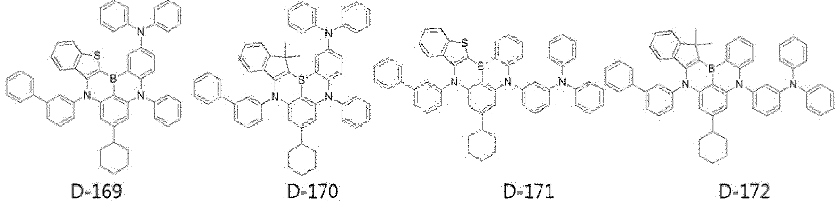

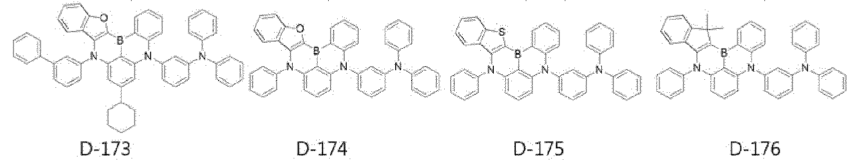

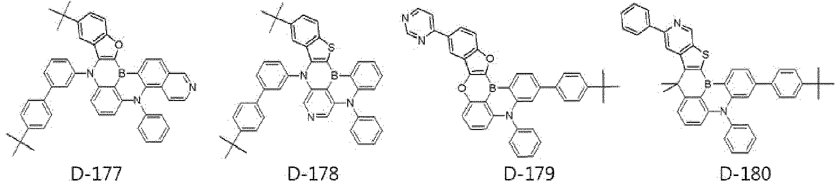

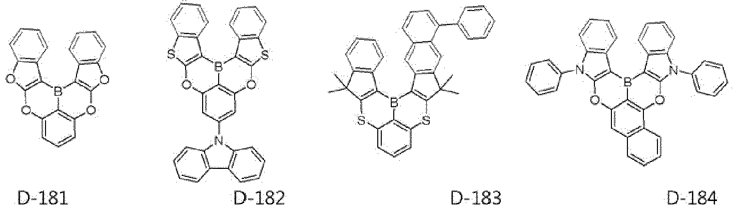

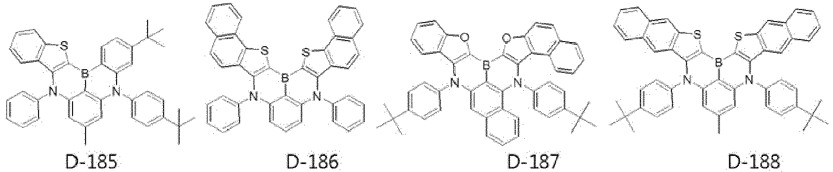

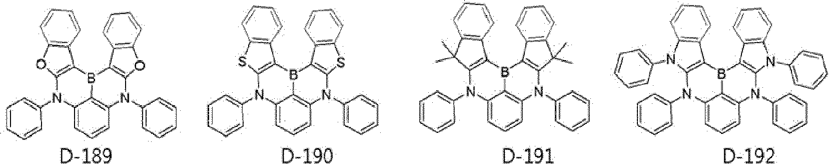

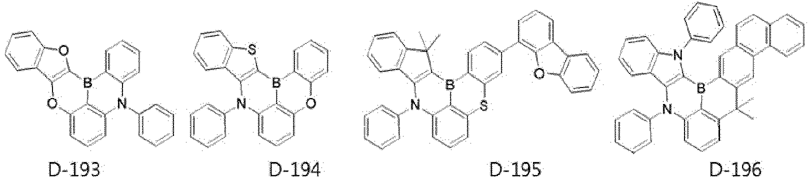

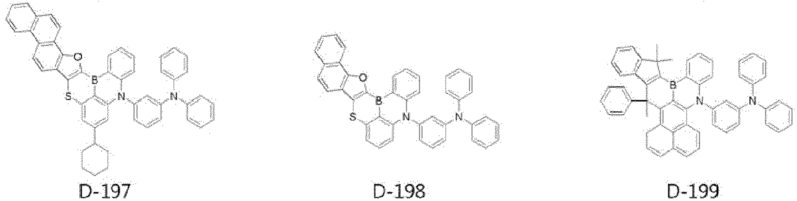

However, these compounds are not intended to limit the scope of Formulas D-1 and D-2.

The organic layers of the organic light emitting device according to the present invention may form a monolayer structure. Alternatively, the organic layers may be stacked together to form a multilayer structure. For example, the organic layers may have a structure including a hole injecting layer, a hole transport layer, a hole blocking layer, a light emitting layer, an electron blocking layer, an electron transport layer, and an electron injecting layer but are not limited to this structure. The number of the organic layers is not limited and may be increased or decreased. Preferred structures of the organic layers of the organic light emitting device according to the present invention will be explained in more detail in the Examples section that follows.

A more detailed description will be given concerning exemplary embodiments of the organic light emitting device according to the present invention.

The organic light emitting device of the present invention includes an anode, a hole transport layer, a light emitting layer, an electron transport layer, and a cathode. The organic light emitting device of the present invention may optionally further include a hole injecting layer between the anode and the hole transport layer and an electron injecting layer between the electron transport layer and the cathode. If necessary, the organic light emitting device of the present invention may further include one or two intermediate layers such as a hole blocking layer or an electron blocking layer. The organic light emitting device of the present invention may further include one or more organic layers such as a capping layer that have various functions depending on the desired characteristics of the device.

A specific structure of the organic light emitting device according to one embodiment of the present invention, a method for fabricating the device, and materials for the organic layers are as follows.

First, an anode material is coated on a substrate to form an anode. The substrate may be any of those used in general organic light emitting devices. The substrate is preferably an organic substrate or a transparent plastic substrate that is excellent in transparency, surface smoothness, ease of handling, and waterproofness. A highly transparent and conductive metal oxide such as indium tin oxide (ITO), indium zinc oxide (IZO), tin oxide (SnO₂) or zinc oxide (ZnO) is used as the anode material.

A hole injecting material is coated on the anode by vacuum thermal evaporation or spin coating to form a hole injecting layer. Then, a hole transport material is coated on the hole injecting layer by vacuum thermal evaporation or spin coating to form a hole transport layer.

The hole injecting material is not specially limited so long as it is usually used in the art. Specific examples of such materials include 4,4',4"-tris(2-naphthylphenyl-phenylamino)triphenylamine (2-TNATA), N,N'-di(1-naphthyl)-N,N'-diphenylbenzidine (NPD), N,N' -diphenyl-N,N' -bis(3 -methylphenyl)-1,1' -biphenyl-4,4' -diamine (TPD), and N,N'-diphenyl-N,N'-bis(4-(phenyl-m-tolylamino)phenyl)biphenyl-4,4'-diamine (DNTPD).

The hole transport material is not specially limited so long as it is commonly used in the art. Examples of such materials include N,N'-bis(3-methylphenyl)-N,N'-diphenyl-(1,1-biphenyl)-4,4'-diamine (TPD) and N,N'-di(naphthalen-1-yl)-N,N'-diphenylbenzidine (α-NPD).

Subsequently, a hole auxiliary layer and a light emitting layer are sequentially laminated on the hole transport layer. A hole blocking layer may be optionally formed on the light emitting layer by vacuum thermal evaporation or spin coating. The hole blocking layer is formed as a thin film and blocks holes from entering a cathode through the organic light emitting layer. This role of the hole blocking layer prevents the lifetime and efficiency of the device from deteriorating. A material having a very low highest occupied molecular orbital (HOMO) energy level is used for the hole blocking layer. The hole blocking material is not particularly limited so long as it can transport electrons and has a higher ionization potential than the light emitting compound. Representative examples of suitable hole blocking materials include BAlq, BCP, and TPBI.

Examples of materials for the hole blocking layer include, but are not limited to, BAlq, BCP, Bphen, TPBI, TAZ, BeBq₂, OXD-7, and Liq.

An electron transport layer is deposited on the hole blocking layer by vacuum thermal evaporation or spin coating, and an electron injecting layer is formed thereon. A cathode metal is deposited on the electron injecting layer by vacuum thermal evaporation to form a cathode, completing the fabrication of the organic light emitting device.

For example, lithium (Li), magnesium (Mg), aluminum (Al), aluminum-lithium (Al-Li), calcium (Ca), magnesium-indium (Mg-In) or magnesium-silver (Mg-Ag) may be used as the metal for the formation of the cathode. The organic light emitting device may be of top emission type. In this case, a transmissive material such as ITO or IZO may be used to form the cathode.

A material for the electron transport layer functions to stably transport electrons injected from the cathode. The electron transport material may be any of those known in the art and examples thereof include, but are not limited to, quinoline derivatives, particularly tris(8-quinolinolate)aluminum (Alq3), TAZ, Balq, beryllium bis(benzoquinolin-10-olate) (Bebq2), ADN, and oxadiazole derivatives such as PBD, BMD, and BND.

Each of the organic layers can be formed by a monomolecular deposition or solution process. According to the monomolecular deposition process, the material for each layer is evaporated into a thin film under heat and vacuum or reduced pressure. According to the solution process, the material for each layer is mixed with a suitable solvent and the mixture is then formed into a thin film by a suitable method such as inkjet printing, roll-to-roll coating, screen printing, spray coating, dip coating or spin coating.

The organic light emitting device of the present invention can be used in a display or lighting system selected from flat panel displays, flexible displays, monochromatic flat panel lighting systems, white flat panel lighting systems, flexible monochromatic lighting systems, flexible white lighting systems, displays for automotive applications, displays for virtual reality, and displays for augmented reality.

### Mode for Carrying out the Invention

The present invention will be more specifically explained with reference to the following examples. However, it will be obvious to those skilled in the art that these examples are in no way intended to limit the scope of the invention.

### Synthesis Example 1. Synthesis of 1

### Synthesis Example 1-1: Synthesis of 1-a

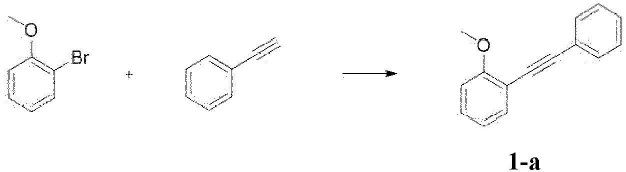

2-Bromoanisole (30 g, 0.171 mol), phenylacetylene (38.5 g, 0.377 mol), bis(triphenylphosphine)palladium(II) dichloride (2.41 g, 0.003 mol), copper(I) iodide (1.63 g, 0.009 mol), and triphenylphosphine (0.45 g, 0.002 mol) were added to 300 mL of triethylamine in a 1 L reactor. The mixture was stirred under reflux overnight. After completion of the reaction, the reaction mixture was allowed to cool to room temperature and filtered with hexane. The filtrate was concentrated and purified by column chromatography to afford **1-a** (25 g, 74%).

### Synthesis Example 1-2: Synthesis of 1-b

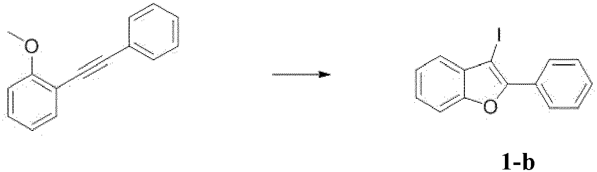

**1-a** (25 g, 0.120 mol) was dissolved in dichloromethane in a 1 L reactor and purged with nitrogen. To the resulting solution was added iodine (45.7 g, 0.180 mol). The mixture was stirred at room temperature for 12 h. After completion of the reaction, the reaction mixture was added with an aqueous sodium thiosulfate solution to remove residual iodine, extracted with dichloromethane, and purified by column chromatography to afford **1-b** (31 g, 81%).

### Synthesis Example 1-3: Synthesis of 1

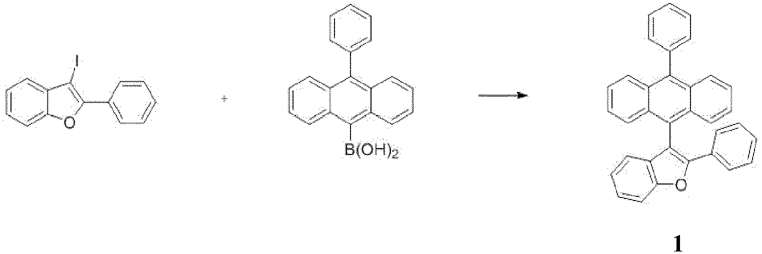

**1-b** (31 g, 0.097 mol), 10-phenylanthracene-9-boronic acid (31.7 g, 0.107 mol), potassium carbonate (26.8 g, 0.194 mol), tetrakis(triphenylphosphine)palladium(0) (2.24 g, 0.002 mol), 210 mL of toluene, and 90 mL of ethanol were placed in a 1 L reactor. The mixture was stirred under reflux at 110 °C overnight. The reaction mixture was allowed to cool to room temperature and extracted with ethyl acetate/distilled water. The organic layer was concentrated and purified by column chromatography to afford 1 (19 g, 44%).
MS (MALDI-TOF): m/z 446.17 [M⁺]

### Synthesis Example 2. Synthesis of 8

### Synthesis Example 2-1: Synthesis of 2-a

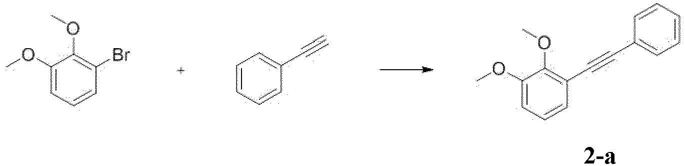

**2-a** (yield 79%) was synthesized in the same manner as in Synthesis Example 1-1, except that 3-bromoveratrol was used instead of 2-bromoanisole.

### Synthesis Example 2-2: Synthesis of 2-b

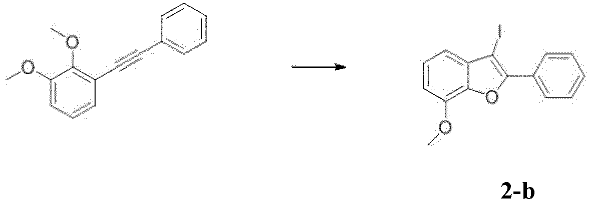

**2-b** (yield 63%) was synthesized in the same manner as in Synthesis Example 1-2, except that **2-a** was used instead of **1-a.**

### Synthesis Example 2-3: Synthesis of 2-c

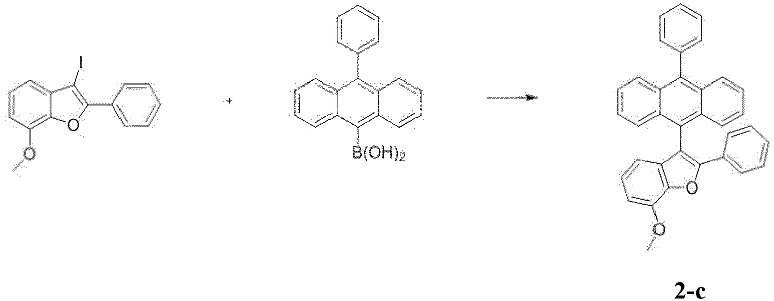

**2-c** (yield 61%) was synthesized in the same manner as in Synthesis Example 1-3, except that **2-b** was used instead of **1-b.**

### Synthesis Example 2-4: Synthesis of 2-d

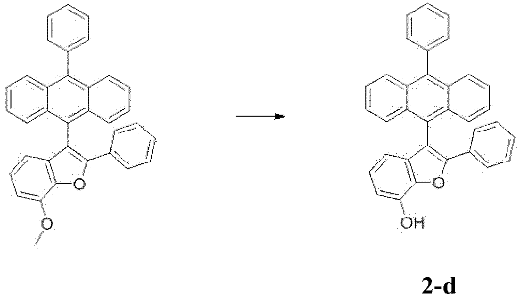

**2-c** (20 g, 0.042 mol) was dissolved in dichloromethane in a 500 mL reactor. After the temperature of the reactor was lowered to 0 °C, BBr₃ (15.7 g, 0.063 mol) was slowly added to the solution. Thereafter, the temperature was raised to room temperature. The mixture was stirred for 2 h. Cold distilled water was slowly added to quench the reaction. The reaction mixture was extracted with dichloromethane and distilled water. The organic layer was concentrated and purified by column chromatography to afford **2-d** (17 g, 87%).

### Synthesis Example 2-5: Synthesis of 2-e

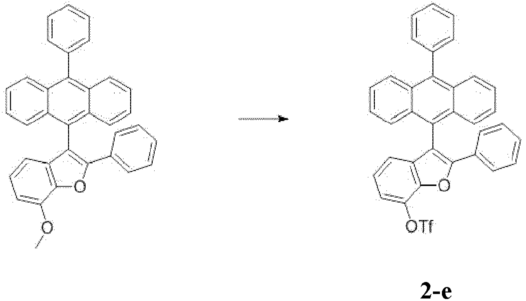

**2-d** (17 g, 0.037 mol) was dissolved in 170 mL of dichloromethane in a 500 mL reactor. After the temperature of the reactor was lowered to 0 °C, trifluoromethanesulfonic anhydride (12.4 g, 0.044 mol) was slowly added to the solution. Thereafter, the temperature was raised to room temperature. The mixture was stirred for 3 h. Cold distilled water was slowly added to quench the reaction. The reaction mixture was extracted with dichloromethane and distilled water. The organic layer was concentrated and purified by column chromatography to afford **2-e** (18 g, 82%).

### Synthesis Example 2-6: Synthesis of 8

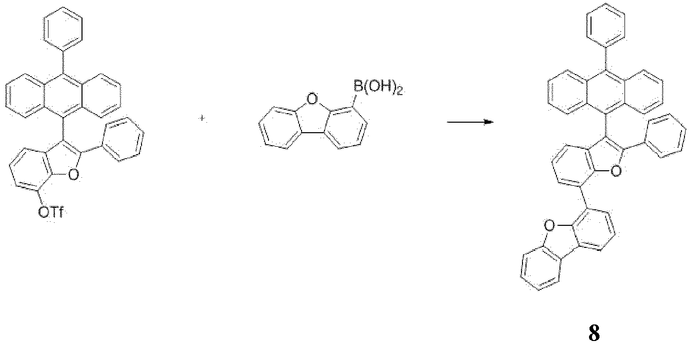

**8** (yield 47%) was synthesized in the same manner as in Synthesis Example 1-3, except that **2-e** and 4-dibenzofuran boronic acid were used instead of **1-b** and 10-phenylanthracene-9-boronic acid, respectively.
MS (MALDI-TOF): m/z 612.21 [M⁺]

### Synthesis Example 3. Synthesis of 14

### Synthesis Example 3-1: Synthesis of 3-a

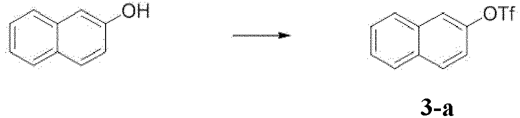

Naphthalen-2-ol (50 g, 0.347 mol) and 500 mL of dichloromethane were stirred in a 1 L reactor and pyridine (20.8 g, 0.867 mol) was added dropwise thereto. Thereafter, the reaction temperature was adjusted to 0 °C. Trifluoromethanesulfonic anhydride (117.4 g, 0.416 mol) was added dropwise to the mixture and then the temperature was raised to room temperature. After completion of the reaction, the reaction mixture was added dropwise with 300 mL of distilled water and extracted with ethyl acetate/distilled water. The organic layer was concentrated and purified by column chromatography to afford **3-a** (80 g, 83%).

### Synthesis Example 3-2: Synthesis of 3-b

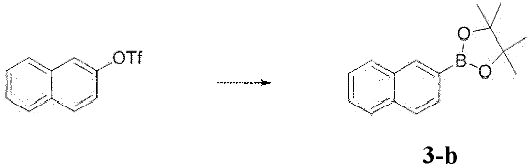

**3-a** (80 g, 0.555 mol), bis(pinacolato)diboron (169.1 g, 0.666 mol), (1,1'-bis(diphenylphosphino)ferrocene)dichloropalladium(II) (13.6 g, 0.017 mol), potassium acetate (163.4 g, 1.665 mol), and 800 mL of dioxane were stirred under reflux in a 1 L reactor. After the completion of the reaction was confirmed by TLC, the reaction solution was filtered. The filtrate was collected, concentrated, and purified by column chromatography to afford Intermediate **3-b** (110 g, 71%).

### Synthesis Example 3-3: Synthesis of 3-c

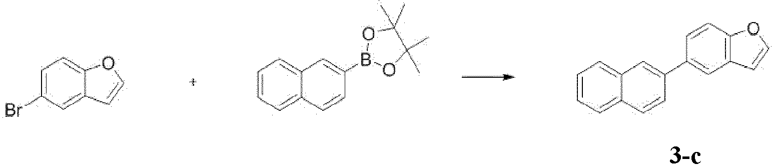

5-Bromobenzofuran (50 g, 0.254 mol), **3-b** (70.94 g, 0.279 mol), tetrakis(triphenylphosphine)palladium(0) (5.86 g, 0.005 mol), potassium carbonate (70.15 g, 0.508 mol), 350 mL of toluene, 150 mL of ethanol, and 100 mL of distilled water were placed in a 500 mL reactor. The mixture was stirred under reflux for 12 h. The reaction mixture was cooled to room temperature and extracted with ethyl acetate/distilled water. The organic layer was concentrated under reduced pressure and purified by column chromatography to afford **3-c** (50 g, 80%).

### Synthesis Example 3-4: Synthesis of 3-d

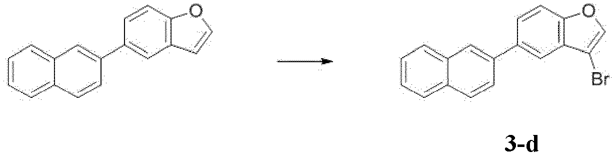

**3-c** (50 g, 0.205 mol) and 1 L of dichloromethane were placed in a 2 L reactor. The mixture was cooled to -10 °C and bromine (36 g, 0.225 mol) was slowly added dropwise thereto. After the completion of the reaction was confirmed by TLC, the temperature was raised to room temperature and an aqueous sodium thiosulfate solution was added dropwise to quench the reaction. The organic layer of the reaction solution was concentrated and 400 mL of ethanol was added dropwise thereto. The mixture was cooled to -10 °C and potassium hydroxide (22.96 g, 0.409 mol) was added dropwise thereto. The resulting mixture was refluxed. After 1 h, the reaction mixture was cooled to room temperature and extracted with ethyl acetate/distilled water. The organic layer was concentrated under reduced pressure and purified by column chromatography to afford **3-d** (40 g, 60%).

### Synthesis Example 3-5: Synthesis of 14

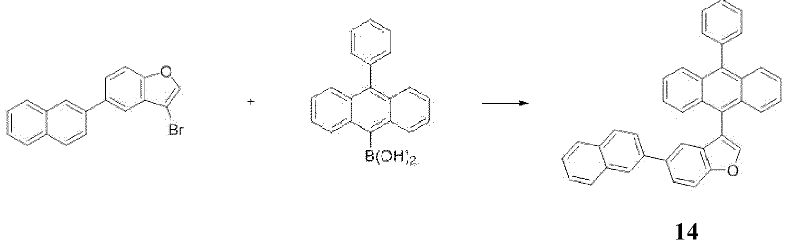

**3-d** (20 g, 0.062 mol), 10-phenyl-anthracene-9-boronic acid (20.3 g, 0.068 mol), palladium(II) acetate (0.28 g, 0.001 mol), potassium carbonate (17.1 g, 0.124 mol), and Sphos (1.02 g, 0.002 mol) were placed in a 500 mL reactor, and then 140 mL of toluene, 60 mL of ethanol, and 40 mL of distilled water were added thereto. The mixture was stirred under reflux at 90 °C for 12 h. The reaction mixture was extracted with ethyl acetate/distilled water. The organic layer was concentrated under reduced pressure, dissolved in toluene, and hot filtered. The filtrate was concentrated, slurried with acetone, filtered, and recrystallized from dichloromethane/acetone to afford **14** (8 g, 26%).
MS (MALDI-TOF): m/z 496.18 [M⁺]

### Synthesis Example 4. Synthesis of 45

**45** (yield 42%) was synthesized in the same manner as in Synthesis Example 1, except that 10-phenyl(d5)-anthracene-9-boronic acid was used instead of 10-phenylanthracene-9-boronic acid in Synthesis Example 1-3.
MS (MALDI-TOF): m/z 451.20 [M⁺]

### Synthesis Example 5. Synthesis of 48

### Synthesis Example 5-1: Synthesis of 5-a

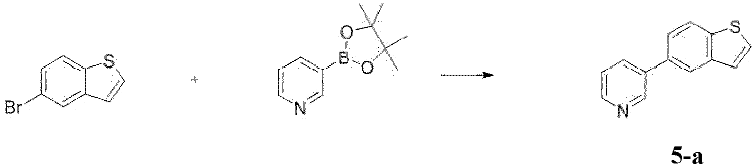

**5-a** (yield 78%) was synthesized in the same manner as in Synthesis Example 3-3, except that 5-bromobenzothiophene and 3-(4,4,5,5-tetramethyl-1,3,2-dioxaborolan-2-yl)pyridine were used instead of 5-bromobenzofuran and **3-b,** respectively.

### Synthesis Example 5-2: Synthesis of 5-b

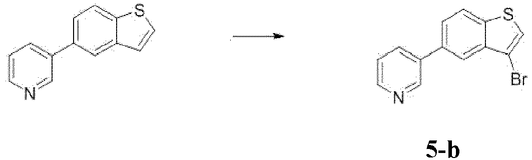

**5-b** (yield 58%) was synthesized in the same manner as in Synthesis Example 3-4, except that **5-a** was used instead of **3-c.**

### Synthesis Example 5-3: Synthesis of 48

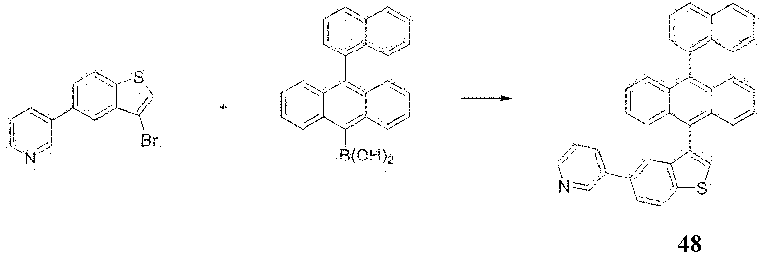

**48** (yield 24%) was synthesized in the same manner as in Synthesis Example 3-5, except that **5-b** and 10-naphthyl-anthracene-9-boronic acid were used instead of **3-d** and 10-phenyl-anthracene-9-boronic acid, respectively.
MS (MALDI-TOF): m/z 513.16 [M⁺]

### Synthesis Example 6. Synthesis of 53

### Synthesis Example 6-1: Synthesis of 6-a

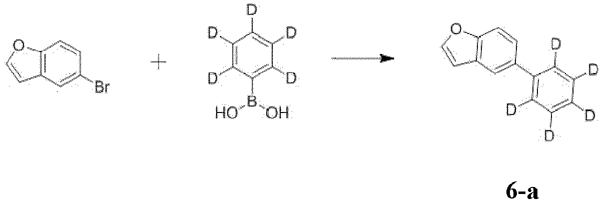

5-Bromobenzofuran (30 g, 0.152 mol), (phenyl-d5)boronic acid (23.2 g, 0.183 mol), tetrakis(triphenylphosphine)palladium(0) (5.3 g, 0.005 mol), potassium carbonate (42.1 g, 0.305 mol), 300 mL of THF, and 120 mL of distilled water were placed in a 500 mL reactor. The mixture was stirred under reflux for 12 h. After completion of the reaction, the reaction mixture was allowed to stand for layer separation. The organic layer was concentrated under reduced pressure and purified by column chromatography to afford **6-a** (21.2 g, 70%).

### Synthesis Example 6-2: Synthesis of 6-b

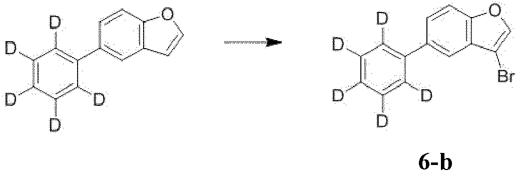

**6-a** (21.2 g, 0.106 mol) and dichloromethane were placed in a 500 mL reactor. The mixture was cooled to -10 °C and bromine was added thereto. The resulting mixture was stirred for 1 h. To the reaction solution was added an aqueous sodium thiosulfate solution, followed by stirring. The mixture was allowed to stand for layer separation. The organic layer was concentrated under reduced pressure. The concentrate was added with ethanol, cooled to -10 °C, and added with an ethanolic solution of potassium hydroxide. The mixture was heated to reflux for 4 h. After completion of the reaction, the reaction mixture was allowed to stand for layer separation. The organic layer was concentrated under reduced pressure and purified by column chromatography to afford **6-b** (20 g, 70%).

### Synthesis Example 6-3: Synthesis of 6-c

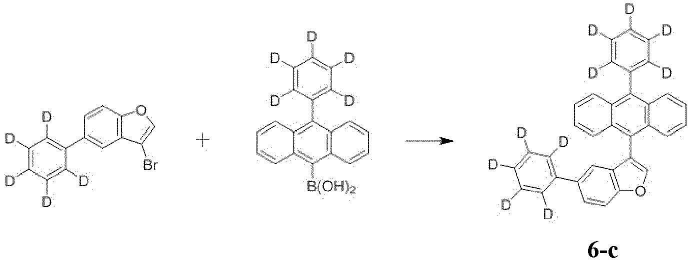

**6-c** (yield 32%) was synthesized in the same manner as in Synthesis Example 3-5, except that **6-b** and (10-(phenyl-d₅)-9-anthracenyl)boronic acid were used instead of **3-d** and 10-phenyl-anthracene-9-boronic acid, respectively.

### Synthesis Example 6-4: Synthesis of 6-d

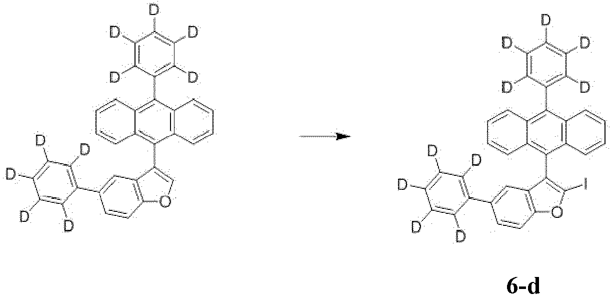

**6-c** (20 g, 0.044 mol) and 250 mL of THF were placed in a 500 mL reactor. The mixture was cooled to -50 °C and n-butyllithium (1.6 M) was added thereto. After 1 h, iodine was slowly added and the temperature was slowly raised to room temperature. After addition of an aqueous sodium thiosulfate solution at room temperature, the resulting mixture was allowed to stand for layer separation. The organic layer was concentrated under reduced pressure and purified by column chromatography to afford **6-d** (16 g, 63%).

### Synthesis Example 6-5: Synthesis of 53

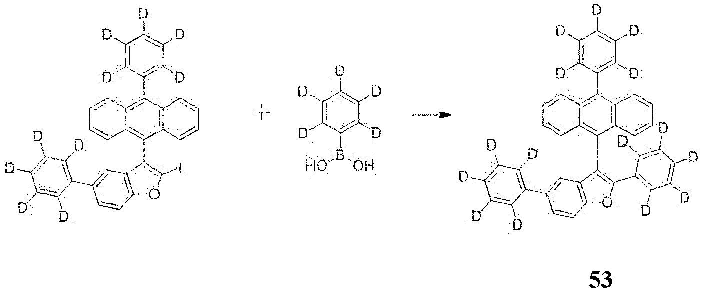

**53** (yield 22%) was synthesized in the same manner as in Synthesis Example 3-5, except that **6-d** and (phenyl-d₅)boronic acid were used instead of **3-d** and 10-phenyl-anthracene-9-boronic acid, respectively.
MS (MALDI-TOF): m/z 537.29 [M⁺]

### Synthesis Example 7. Synthesis of 55

### Synthesis Example 7-1: Synthesis of 55

**55** (yield 25%) was synthesized in the same manner as in Synthesis Example 1, except that B-(10-(1,1'-biphenyl)-2-yl-9-anthracenyl)boronic acid was used instead of 10-phenylanthracene-9-boronic acid in Synthesis Example 1-3.
MS (MALDI-TOF): m/z 522.20 [M⁺]

### Examples 1 to 7: Fabrication of organic light emitting devices

ITO glass was patterned to have a light emitting area of 2 mm × 2 mm, followed by cleaning. After the cleaned ITO glass was mounted in a vacuum chamber, the base pressure was adjusted to 1 × 10⁻⁷ torr. DNTPD (700 Å) and α-NPD (300 Å) were sequentially formed into layers on the ITO. A mixture of the inventive host compound and the dopant compound (3 wt%) shown in Table 1 was formed into a 300 Å thick light emitting layer. Thereafter, a mixture of the compound represented by Formula E-1 and the compound represented by Formula E-2 in a ratio of 1:1 was formed into a 300 Å thick electron transport layer on the light emitting layer. The compound represented by Formula E-2 was formed into a 10 Å thick electron injecting layer on the electron transport layer. Al was formed into a 1000 Å thick electrode on the electron injecting layer, completing the fabrication of an organic light emitting device. The luminescent properties of the organic light emitting device were measured at 10 mA/cm².

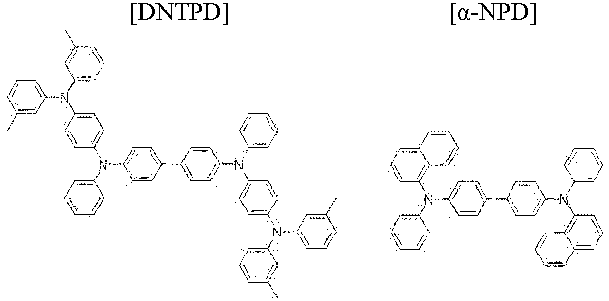

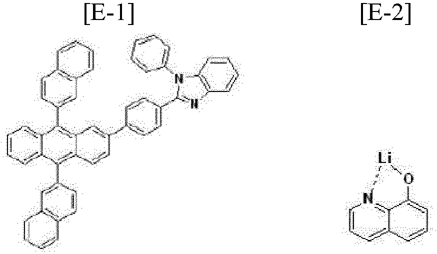

### Comparative Examples 1 to 4

Organic light emitting devices were fabricated in the same manner as in Examples 1-7, except that BH-1, BH-2, BH-3 or BH-4 was used instead of the inventive host compound. The luminescent properties of the organic light emitting devices were measured at 10 mA/cm². The structures of BH-1 to BH-4 are as follow:

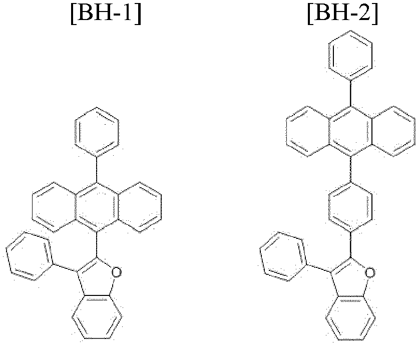

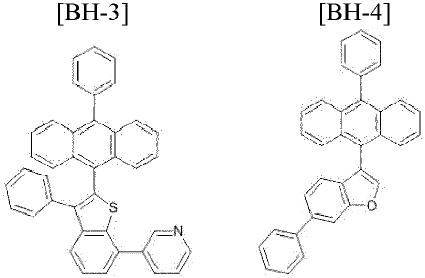

**[Table 1]**

| Example No. | Host | Dopant | Current density (mA/cm²) | External quantum efficiency (EQE, %) |
|---|---|---|---|---|
| Example 1 | 1 | D-116 | 10 | 12.0 |
| Example 2 | 8 | D-116 | 10 | 12.0 |
| Example 3 | 14 | D-116 | 10 | 11.9 |
| Example 4 | 45 | D-116 | 10 | 12.3 |
| Example 5 | 48 | D-116 | 10 | 11.2 |
| Example 6 | 53 | D-116 | 10 | 12.7 |
| Example 7 | 55 | D-116 | 10 | 12.1 |
| Comparative Example 1 | BH-1 | D-116 | 10 | 8.8 |
| Comparative Example 2 | BH-2 | D-116 | 10 | 10.7 |
| Comparative Example 3 | BH-3 | D-116 | 10 | 6.7 |
| Comparative Example 4 | BH-4 | D-116 | 10 | 10.9 |

As can be seen from the results in Table 1, the organic light emitting devices of Examples 1-7, each of which employed the inventive host for the light emitting layer, showed significantly high external quantum efficiencies compared to the organic light emitting devices of Comparative Examples 1-4, each of which employed the host compound whose structural features are contrasted with those of the inventive compound. These results concluded that the use of the inventive compounds makes the organic light emitting devices highly efficient.

### Industrial Applicability

The organic light emitting device of the present invention includes a light emitting layer employing the anthracene derivative with a specific structure as a host. The use of the host ensures excellent luminescent properties (including high luminous efficiency) and high efficiency of the device. Due to these advantages, the organic light emitting device of the present invention can find useful industrial applications in not only lighting systems but also a variety of displays, including flat panel displays, flexible displays, and wearable displays.

## Claims

1. An organic light emitting device comprising a first electrode, a second electrode opposite to the first electrode, and one or more organic layers interposed between the first and second electrodes wherein one of the organic layers is a light emitting layer, wherein the light emitting layer comprises at least one host and at least one dopant, and wherein the host is a compound represented by Formula A: 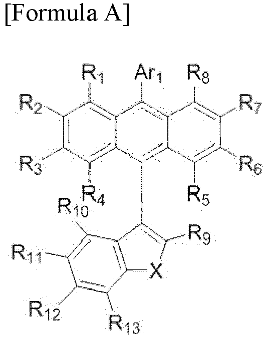 wherein Ar₁ is selected from substituted or unsubstituted C₆-C₅₀ aryl, substituted or unsubstituted C₂-C₅₀ heteroaryl, and substituted or unsubstituted C₃-C₅₀ mixed aliphatic-aromatic cyclic groups, R₁ to R₁₃ are the same as or different from each other and are each independently selected from hydrogen, deuterium, substituted or unsubstituted C₁-C₃₀ alkyl, substituted or unsubstituted C₆-C₅₀ aryl, substituted or unsubstituted C₂-C₃₀ alkenyl, substituted or unsubstituted C₂-C₃₀ alkynyl, substituted or unsubstituted C₃-C₃₀ cycloalkyl, substituted or unsubstituted C₃-C₃₀ cycloalkenyl, substituted or unsubstituted C₂-C₅₀ heteroaryl, substituted or unsubstituted C₂-C₃₀ heterocycloalkyl, substituted or unsubstituted C₁-C₃₀ alkoxy, substituted or unsubstituted C₆-C₅₀ aryloxy, substituted or unsubstituted C₁-C₃₀ alkylthioxy, substituted or unsubstituted C₆-C₅₀ arylthioxy, substituted or unsubstituted amine, substituted or unsubstituted silyl, substituted or unsubstituted germanium, substituted or unsubstituted C₃-C₅₀ mixed aliphatic-aromatic cyclic groups, cyano, and halogen (with the proviso that R₁₂ is other than aryl), and X is an oxygen (O) or sulfur (S) atom, the "substituted" in the definitions of Ar₁ and R₁ to R₁₃ indicating substitution with one or more substituents selected from deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₃₀ aryl, C₇-C₃₀ arylalkyl, C₇-C₃₀ alkylaryl, C₂-C₃₀ heteroaryl, C₂-C₃₀ heteroarylalkyl, C₁-C₂₄ alkoxy, C₁-C₂₄ amine, C₁-C₂₄ silyl, C₁-C₂₄ germanium, C₆-C₃₀ aryloxy, C₃-C₃₀ mixed aliphatic-aromatic cyclic groups, boron, aluminum, phosphoryl, hydroxyl, selenium, tellurium, nitro, and halogen and one or more hydrogen atoms in each of the substituents being optionally replaced by deuterium atoms.

2. The organic light emitting device according to claim 1, wherein R₉ in Formula A is substituted or unsubstituted C₆-C₃₀ aryl.

3. The organic light emitting device according to claim 3, wherein at least one of R₁₀, R₁₁, and R₁₂ is substituted or unsubstituted C₆-C₃₀ aryl.

4. The organic light emitting device according to claim 1, wherein the compound represented by Formula A is selected from the following compounds 1 to 60:

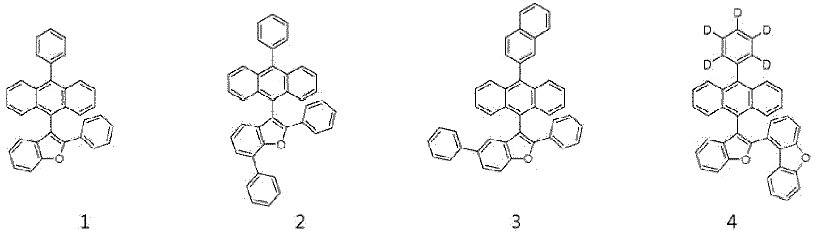

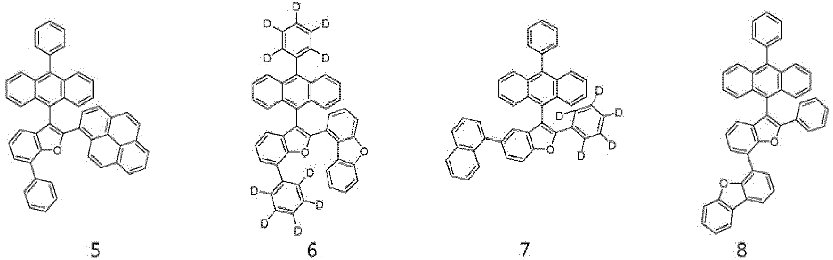

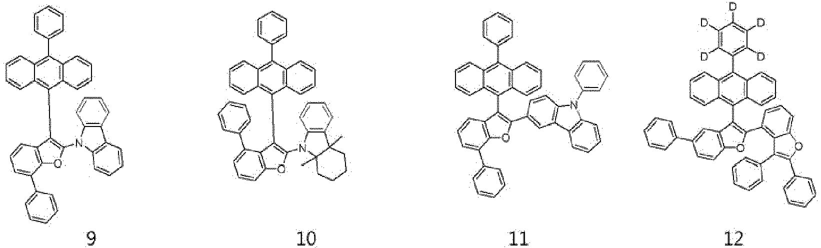

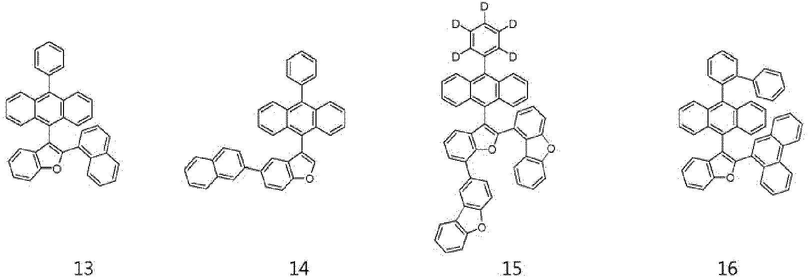

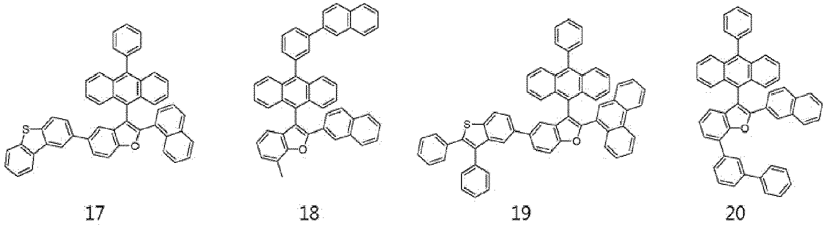

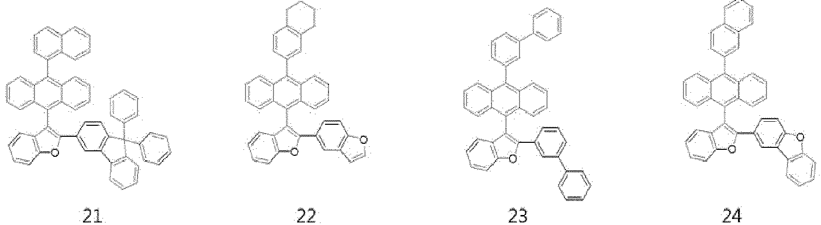

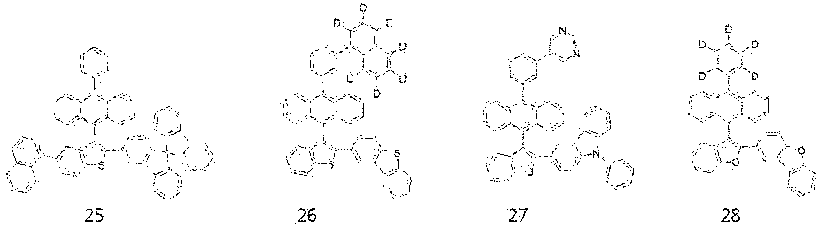

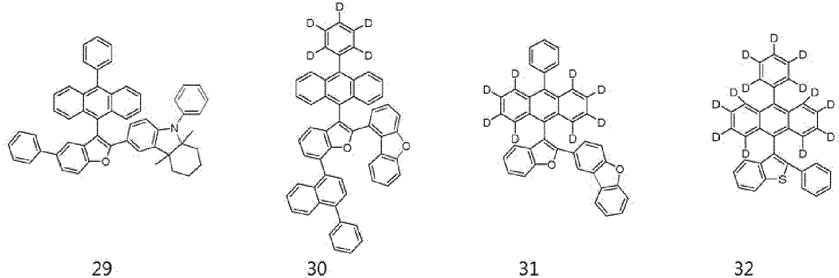

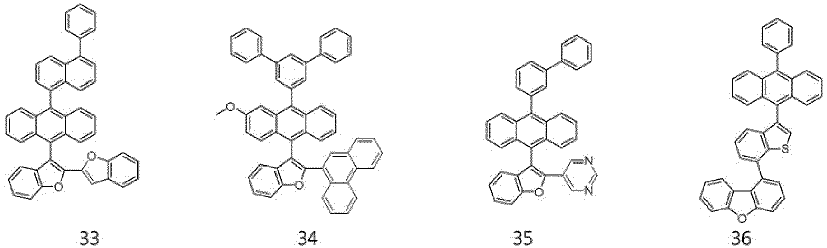

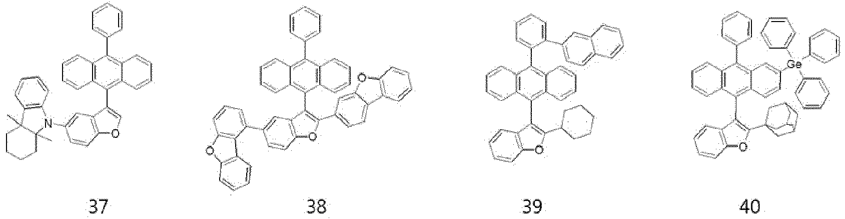

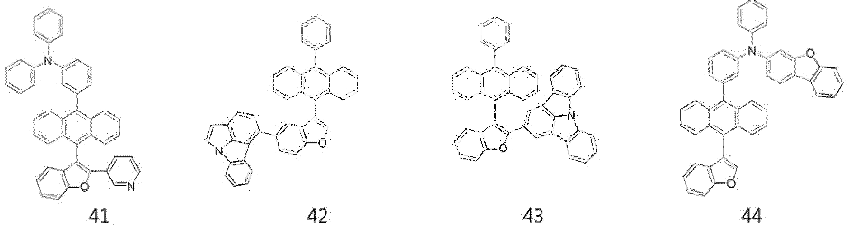

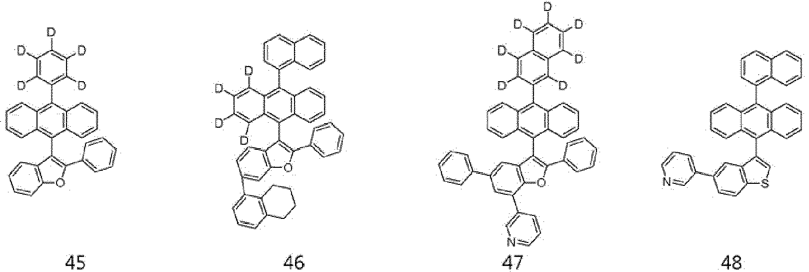

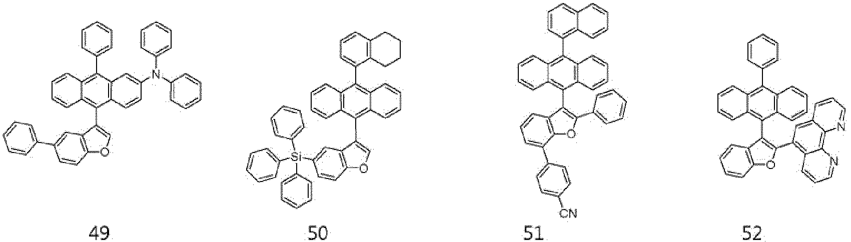

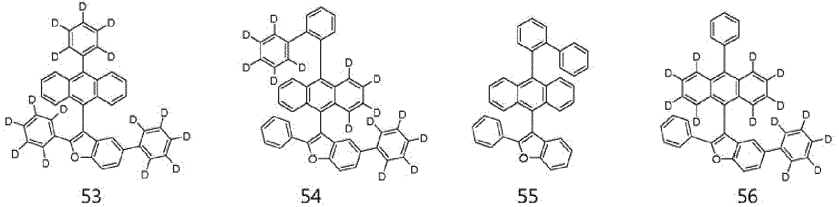

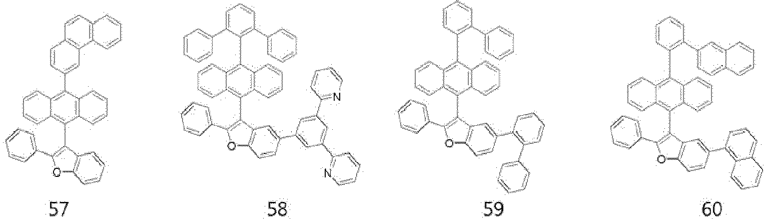

5. The organic light emitting device according to claim 1, wherein the organic layers comprise a hole injecting layer, a hole transport layer, a functional layer having functions of both hole injection and hole transport, an electron transport layer, an electron injecting layer, and/or a functional layer having functions of both electron injection and electron transport.

6. The organic light emitting device according to claim 1, wherein one or more hosts other than the compound represented by Formula A are mixed or stacked in the light emitting layer.

7. The organic light emitting device according to claim 1, wherein the dopant is selected from compounds represented by Formulas D-1 and D-2: 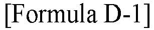 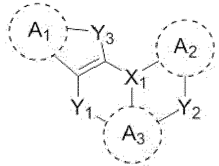 wherein X₁ is selected from B, P=O, and P=S, Y₁ to Y₃ are each independently selected from NR₂₁, CR₂₂R₂₃, O, S, Se, and SiR₂₄R₂₅, A₁ to A₃ are each independently selected from substituted or unsubstituted C₆-C₅₀ aromatic hydrocarbon rings, substituted or unsubstituted C₂-C₅₀ aromatic heterocyclic rings, substituted or unsubstituted C₃-C₃₀ aliphatic rings, and substituted or unsubstituted C₃-C₃₀ mixed aliphatic-aromatic rings, and R₂₁ to R₂₅ are the same as or different from each other and are each independently selected from hydrogen, deuterium, substituted or unsubstituted C₁-C₃₀ alkyl, substituted or unsubstituted C₂-C₃₀ alkenyl, substituted or unsubstituted C₂-C₃₀ alkynyl, substituted or unsubstituted C₆-C₅₀ aryl, substituted or unsubstituted C₃-C₃₀ cycloalkyl, substituted or unsubstituted C₃-C₃₀ heterocycloalkyl, substituted or unsubstituted C₂-C₅₀ heteroaryl, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₆-C₃₀ arylthioxy, substituted or unsubstituted amine, substituted or unsubstituted silyl, substituted or unsubstituted C₃-C₂₀ mixed aliphatic-aromatic cyclic groups, nitro, cyano, and halogen, with the proviso that each of R₂₁ to R₂₅ is optionally bonded to one or more of the rings A₁ to A₃ to form an alicyclic or aromatic monocyclic or polycyclic ring, the substituents of each of the rings A₁ to A₃ are optionally linked to each other to form an alicyclic or aromatic monocyclic or polycyclic ring, R₂₂ and R₂₃ are optionally linked to each other to form an alicyclic or aromatic monocyclic or polycyclic ring, and R₂₄ and R₂₅ are optionally linked to each other to form an alicyclic or aromatic monocyclic or polycyclic ring, and 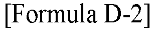 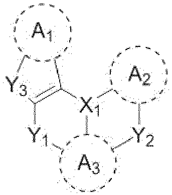 wherein X₁, Y₁ to Y₃, and A₁ to A₃ are as defined in Formula D-1, the "substituted" in the definitions of A₁ to A₃ and R₂₁ to R₂₅ indicating substitution with one or more substituents selected from deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₃₀ aryl, C₇-C₃₀ arylalkyl, C₇-C₃₀ alkylaryl, C₂-C₃₀ heteroaryl, C₂-C₃₀ heteroarylalkyl, C₁-C₂₄ alkoxy, C₁-C₂₄ amine, C₁-C₂₄ silyl, C₆-C₃₀ aryloxy, C₃-C₃₀ mixed aliphatic-aromatic cyclic groups, boron, aluminum, phosphoryl, hydroxyl, selenium, tellurium, nitro, and halogen and one or more hydrogen atoms in each of the substituents being optionally replaced by deuterium atoms.

8. The organic light emitting device according to claim 7, wherein one or more dopants other than the compound represented by Formula D-1 or D-2 are mixed or stacked in the light emitting layer.

9. The organic light emitting device according to claim 7, wherein the compounds represented by Formulas D-1 and D-2 are selected from the following compounds D-101 to D-199:

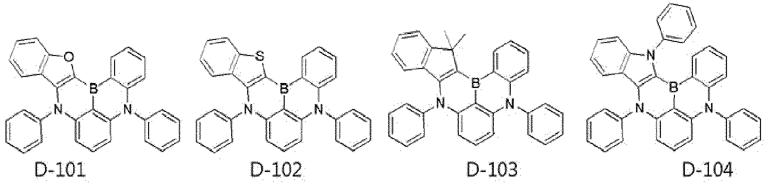

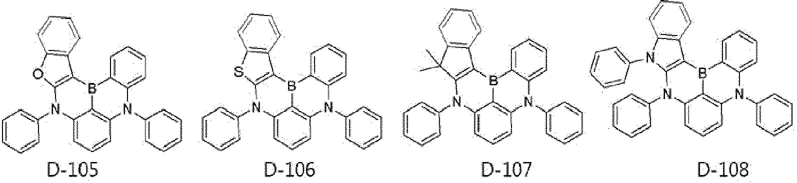

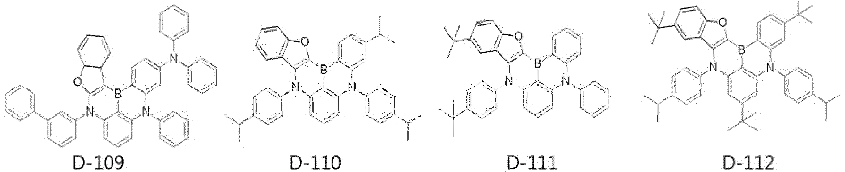

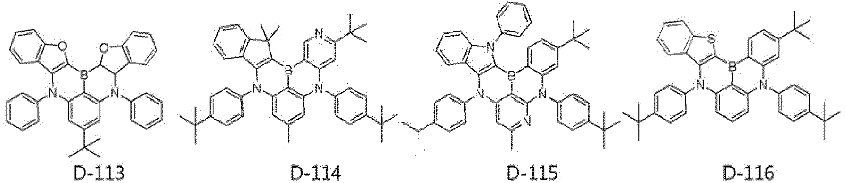

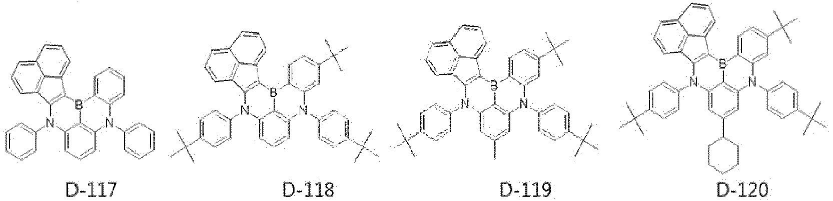

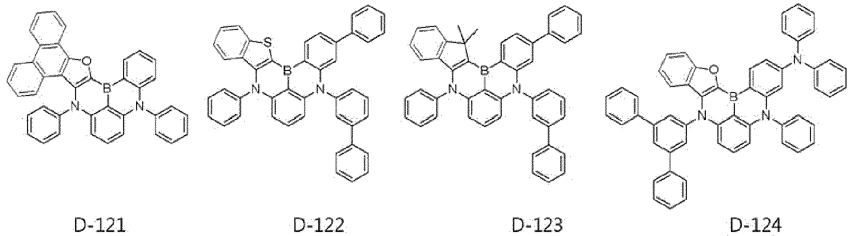

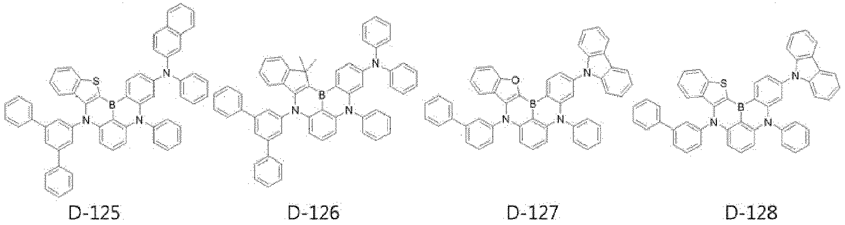

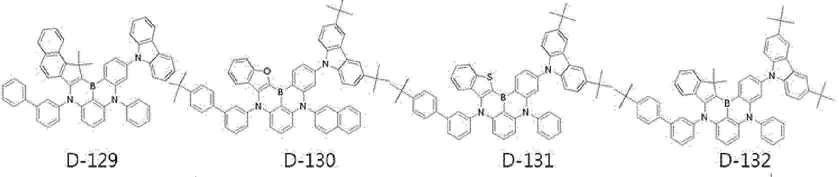

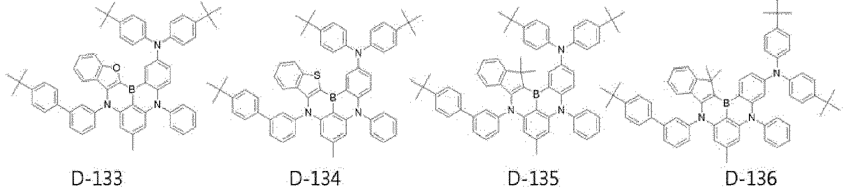

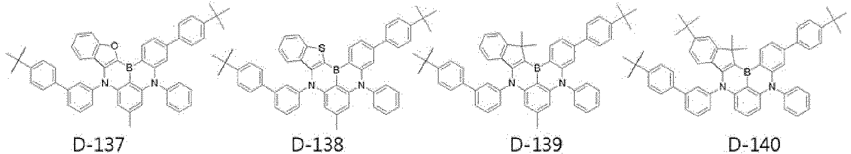

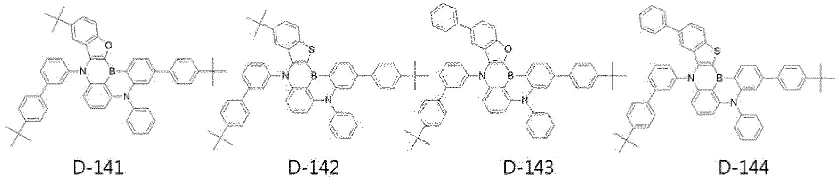

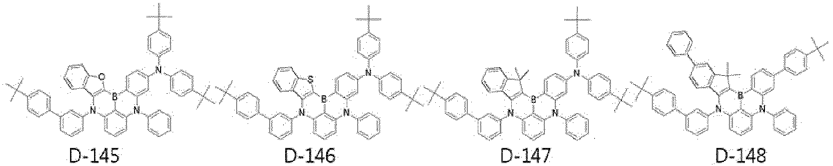

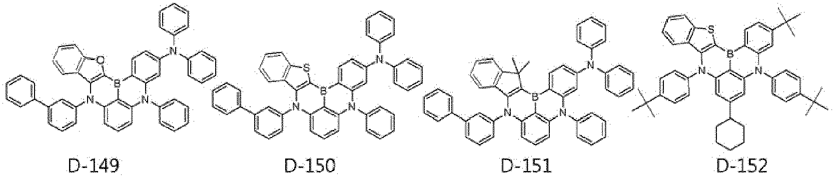

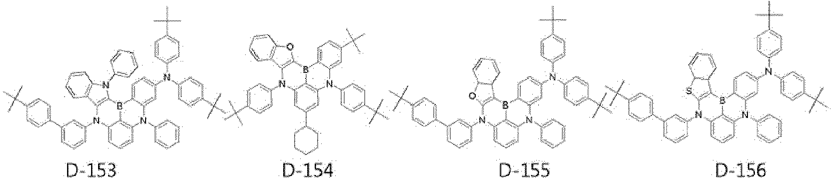

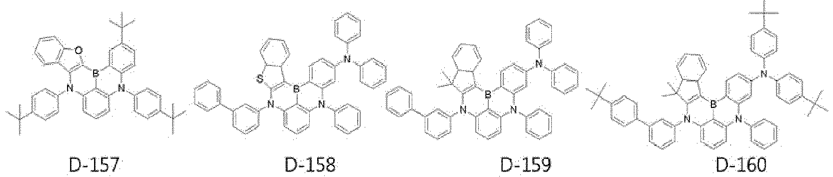

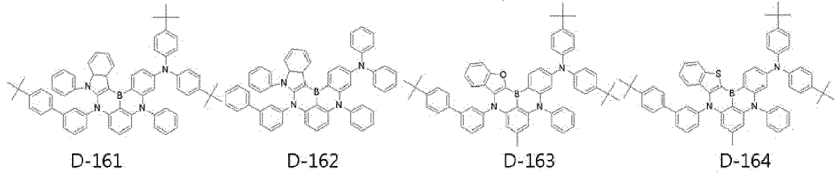

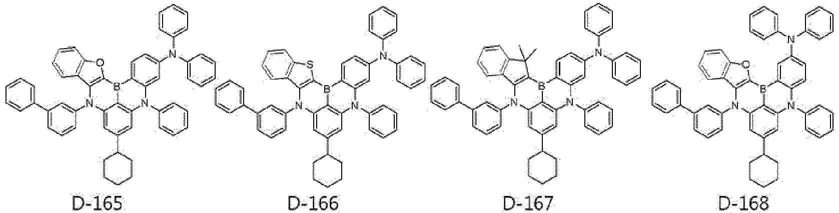

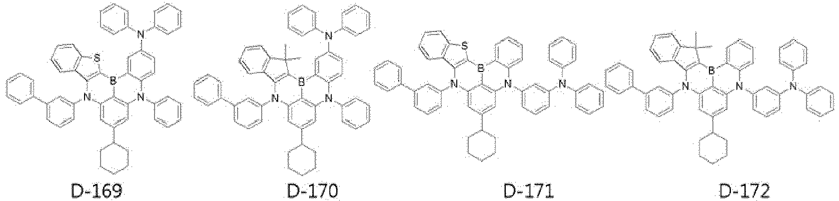

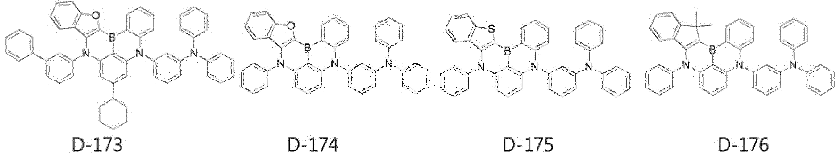

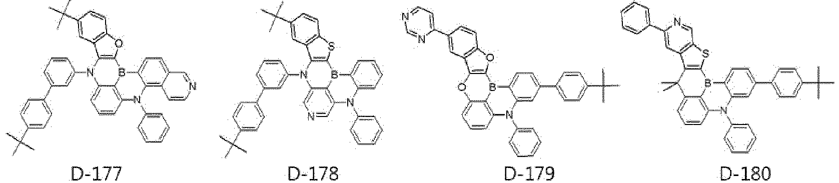

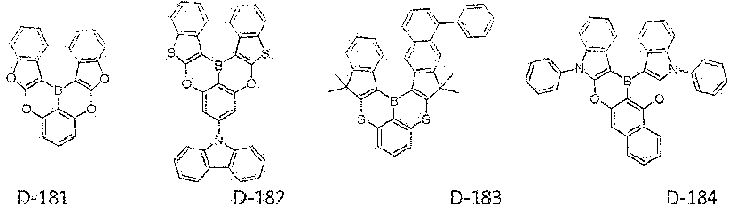

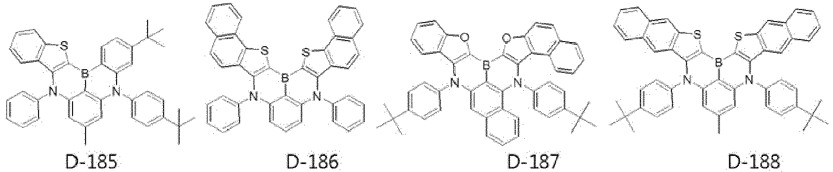

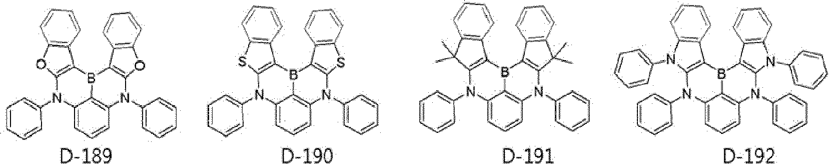

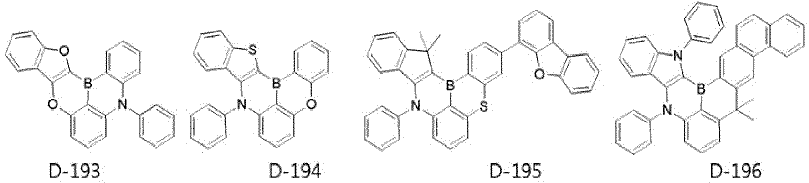

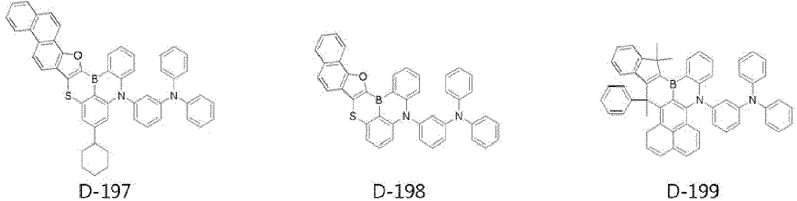

10. The organic light emitting device according to claim 5, wherein one or more of the layers are formed by a deposition or solution process.

11. The organic light emitting device according to claim 1, wherein the organic light emitting device is used in a display or lighting system selected from flat panel displays, flexible displays, monochromatic flat panel lighting systems, white flat panel lighting systems, flexible monochromatic lighting systems, flexible white lighting systems, displays for automotive applications, displays for virtual reality, and displays for augmented reality.
